# EUROPEAN PATENT SPECIFICATION

(11) **EP 1 241 179 B1**
(45) Date of publication and mention of the grant of the patent: **02.08.2006**
(21) Application number: 02012896.3
(22) Date of filing: 08.03.1999
(51) Int. Cl.: C07K 14/47, C07K 19/00, C12N 15/12, C12N 15/62, C07K 16/18

(54) **Human protein having in vitro antiproliferative activity**
Menschliches Protein mit in vitro antiproliferativer Aktivität.
Protéine humaine ayant une activité antiproliférative in vitro

(30) Priority: 15.04.1998 US 81955 P
(43) Date of publication of application: 18.09.2002
(62) Divisional of application: 99912321.9
(73) Proprietor: GENENTECH, INC., South San Francisco, CA 94080-4990 (US)
(72) Inventor: Wood, William I., Hillsborough, CA 94010 (US); Goddard, Audrey, San Francisco, CA 94131 (US); Gurney, Austin, Belmont, CA 94002 (US); Yuan, Jean, San Mateo, CA 94403 (US); Baker, Kevin P., Darnestown, Maryland 20878 (US); Chen, Jian, Princeton, New Jersey 08540 (US)
(74) Representative: Denison, Christopher Marcus

(56) References cited:
- WO-A-99/10363
- WO-A-99/33979
- WO-A-99/43802
- WO-A-99/50405
- US-A- 5 968 744
- DATABASE EMBL [Online] 19 December 1997 (1997-12-19) STRAUSBERG, R.: "ns66e01.r1 NCI_CGAP_Pr22 Homo sapiens cDNA clone IMAGE:1188600 5' similar to SW:CNI_DROME P49858 CORNICHON PROTEIN. ;, mRNA sequence." Database accession no. AA689524 XP002236504
- DATABASE EMBL [Online] 9 April 1998 (1998-04-09) STRAUSBERG, R.: "ok71h07.s1 NCI_CGAP_GC4 Homo sapiens cDNA clone IMAGE:1519453 3', mRNA sequence." Database accession no. AA902726 XP002236505

## Description

### FIELD OF THE INVENTION

The present invention relates generally to the identification and isolation of DNA and to the recombinant production of polypeptides encoded by that DNA.

### BACKGROUND OF THE INVENTION

Extracellular proteins play an important role in the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors; neuropeptides, and hormones) which are, in turn, received and interpreted by diverse cell receptors or membrane-bound proteins. These secreted polypeptides or signaling molecules normally pass through the cellular secretory pathway to reach their site of action in the extracelltilar environment.

Secreted proteins have various industrial applications, including pharmaceuticals, diagnostics, biosensors and bioreactors. Most protein drugs available at present, such as thrombolytic agents, interferons, interleukins, erythropoietins, colony stimulating factors, and various other cytokines, are secretory proteins. Their receptors, which are membrane proteins, also have potential as therapeutic or diagnostic agents. Efforts are being undertaken by both industry and academia to identify new, native secreted proteins. Many efforts are focused on the screening of mammalian recombinant DNA libraries to identify the coding sequences for novel secreted proteins. Examples of screening methods and techniques are described in the literature [see, for example, Klein et al., Proc. Natl. Acad. Sci., 93:7108-7113 (1996); U.S. Patent No. 5,536,637)].

Membrane-bound proteins and receptors can play an important role in the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. Such membrane-bound proteins and cell receptors include, but are not limited to, cytokine receptors, receptor kinases, receptor phosphatases, receptors involved in cell-cell interactions, and cellular adhesin molecules like selectins and integrins. For instance, transduction of signals that regulate cell growth and differentiation is regulated in part by phosphorylation of various cellular proteins. Protein tyrosine kinases, enzymes that catalyze that process, can also act as growth factor receptors. Examples include fibroblast growth factor receptor and nerve growth factor receptor.

Membrane-bound proteins and receptor molecules have various industrial applications, including as pharmaceutical and diagnostic agents. Receptor immunoadhesins, for instance, can be employed as therapeutic agents to block receptor-ligand interaction. The membrane-bound proteins can also be employed for screening of potential peptide or small molecule inhibitors of the relevant receptor/ligand interaction. Efforts are being undertaken by both industry and academia to identify new, native receptor proteins. Many efforts are focused on the screening of mammalian recombinant DNA libraries to identify the coding sequences for novel receptor proteins.

We herein describe the identification and characterization of secreted and transmembrane polypeptides and nucleic acids encoding those polypeptides.

### PRO181

In Drosophila, the dorsal-ventral polarity of the egg chamber depends on the localization of the oocyte nucleus and the gurken RNA to the dorsal-anterior corner of the oocyte. Gurken protein presumably acts as a ligand for the drosophila EGF receptor (torpedo/DER) expressed in the somatic follicle cells surrounding the oocyte. Cornichon is a gene required in the germline for dorsal-ventral signaling (Roth et al., Cell 81:967-978 (1995)). Cornichon, gurken and torpedo also function in an earlier signaling event that establishes posterior follicle cell fates and specifies the anterior-posterior polarity of the egg chamber. Mutations in any or all of these genes prevent the formation of a correctly polarized microtubule cytoskeleton required for proper localization of the anterior and posterior determinants bicoid and oskar and for the asymmetric positioning of the oocyte nucleus. Thus, it is clear that the cornichon gene product plays an important role in early development. We herein describe the identification and characterization of a polypeptide having homology to the cornichon protein, designated herein as PRO181.

WO99/10363, WO99/50405, WO99/43802 and WO99/33979 all report the identification of nucleic acid encoding a polypeptide having homology to cornichon protein.

### SUMMARY OF THE INVENTION

### PR0181

Applicants have identified a cDNA clone that encodes a polypeptide having homology to the cornichon protein, wherein the polypeptide is designated in the present application as "PRO181".

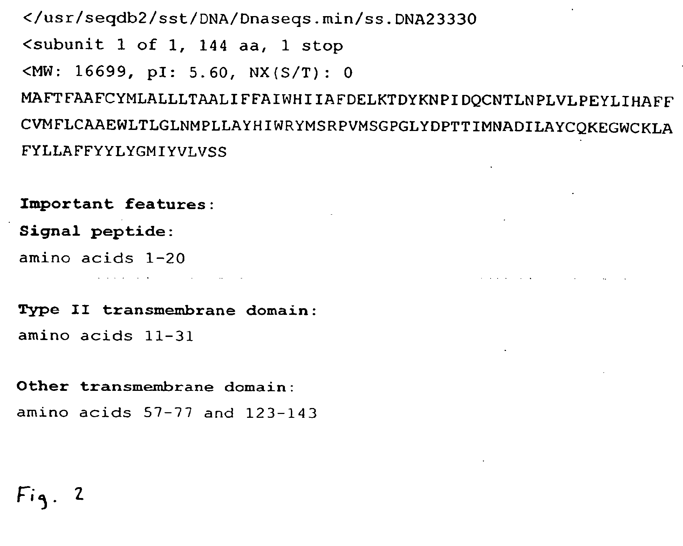

Disclosed herein is an isolated nucleic acid molecule comprising DNA encoding a PRO181 polypeptide. In one aspect, the isolated nucleic acid comprises DNA encoding the PRO181 polypeptide having amino acid residues I to 144 of Figure 2 (SEQ ID NO:2), or is complementary to such encoding nucleic acid sequence, and remains stably bound to it under at least moderate, and optionally, under high stringency conditions. In other aspects, the isolated nucleic acid comprises DNA encoding the PRO181 polypeptide having amino acid residues about 21 to 144 of Figure 2 (SEQ ID NO:2) or amino acid 1 or about 21 to X of Figure 2 (SEQ ID NO:2) where X is any amino acid from 52 to 61 of Figure 2 (SEQ ID NO:2), or is complementary to such encoding nucleic acid sequence, and remains stably bound to it under at least moderate, and optionally, under high stringency conditions. The isolated nucleic acid sequence may comprise the cDNA insert of the DNA23330-1390 vector deposited on April 14, 1998 as ATCC 209775 which includes the nucleotide sequence encoding PRO181.

In one aspect, as defined in the claims, the invention provides isolated PRO 181 polypeptide for use in a method of medical treatment. Further related, aspects of the invention are also defined in the claims. In particular, the invention relates to isolated native sequence PRO181 polypeptide, which in one embodiment, includes an amino acid sequence comprising residues 1 to 144 of Figure 2 (SEQ ID NO:2). Additional embodiments of the present invention are directed to PRO181 polypeptides comprising amino acids about 21 to 144 of Figure 2 (SEQ ID NO:2) or amino acid 1 or about 21 to X of Figure 2 (SEQ ID NO:2), where X is any amino acid from 52 to 61 of Figure 2 (SEQ ID NO:2). Optionally, the PRO181 polypeptide is obtained or is obtainable by expressing the polypeptide encoded by the cDNA insert of the DNA23330-1390 vector deposited on April 14, 1998 as ATCC 209775.

### BRIEF DESCRIPTION OF THE DRAWINGS

Figure 1 shows a nucleotide sequence (SEQ ID NO:1) of a native sequence PRO 181 cDNA, wherein SEQ ID NO:1 is a clone designated herein as "UNQ155" and/or "DNA23330-1390".
Figure 2 shows the amino acid sequence (SEQ ID NO:2) derived from the coding sequence of SEQ ID NO:1 shown in Figure 1.
Figure 3 shows an EST nucleotide sequence designated herein as DNA13242 (SEQ ID NO:3).

### DETAILED DESCRIPTION OF THE PREFERRED EMBODIMENTS

### I. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" as used herein encompass native sequence polypeptides and polypeptide variants (which are further defined herein). The PRO polypeptides described herein may be isolated from a variety of sources, such as from human tissue types or from another source, or prepared by recombinant or synthetic methods.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (*e.g*., an extracellular domain sequence), naturally-occurring variant forms (*e.g*., alternatively spliced forms) and naturally-occurring allelic variants of the polypeptide. In various embodiments of the invention, the native sequence PRO181 polypeptide is a mature or full-length native sequence PRO181 polypeptide comprising amino acids 1 to 144 of Figure 2 (SEQ ID NO:2).

The PRO polypeptide "extracellular domain" or "ECD" refers to a form of the PRO polypeptide which is essentially free of the transmembrane and cytoplasmic domains. Ordinarily, a PRO polypeptide ECD will have less than 1 % of such transmembrane and/or cytoplasmic domains and preferably, will have less than 0.5 % of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified. Optionally, therefore, an extracellular domain of a PRO polypeptide may contain from about 5 or fewer amino acids on either or the transmembrane domain as initially identified.

"PRO polypeptide variant" means an active PRO polypeptide as defined above or below having at least about 80% amino acid sequence identity with the full-length native sequence PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, more preferably at least about 85% amino acid sequence identity, and even more preferably at least about 90% amino acid sequence identity, even more preferably at least about 91% amino acid sequence identity, even more preferably at least about 92% amino acid sequence identity, even more preferably at least about 93% amino acid sequence identity, even more preferably at least about 94% amino acid sequence identity, even more preferably at least about 95% amino acid sequence identity, yet more preferably at least about 96% amino acid sequence identity, yet more preferably at least about 97% amino acid sequence identity, yet more preferably at least about 98% amino acid sequence identity and most preferably at least about 99% amino acid sequence identity with the amino acid sequence of the full-length native amino acid sequence as disclosed herein.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. The preferred software alignment program is BLAST. Those skilled in the art can determine appropriate parameters for measuring alignment, including any algorithms needed to achieve maximal alignment over the full length of the sequences being compared. The % identity values used herein have been generated using the WU-BLAST-2 computer program (Altschul et al., Methods in Enzymology 266:460-480 (1996); http://blast.wustl/edu/blast/README.html). Most of the WU-BLAST-2 search parameters were set to the default values. The adjustable parameters were set with the following values: overlap span = 1, overlap fraction = 0.125, word threshold (T) = 11, and scoring matrix = BLOSUM62. The HSP S and HSP S2 parameters, which are dynamic values used by BLAST-2, are established by the program itself depending upon the composition of the sequence of interest and composition of the database against which the sequence is being searched. However, the values may be adjusted to increase sensitivity. A % sequence identity value is determined by the fraction of matching identical residues divided by the total number of residues in the aligned region.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. The identity values used herein were generated by the BLASTN module of WU-BLAST-2 set to the default parameters, with overlap span and overlap fraction set to 1 and 0.125, respectively.

The term "positives", in the context of sequence comparison performed as described above, includes residues in the sequences compared that are not identical but have similar properties (e.g. as a result of conservative substitutions). The % value of positives is determined by the fraction of residues scoring a positive value in the BLOSUM 62 matrix divided by the total number of residues in the aligned region, as defined above.

The term "epitope tagged" where used herein refers to a chimeric polypeptide comprising a PRO polypeptide, or domain sequence thereof, fused to a "tag polypeptide". The tag polypeptide has enough residues to provide an epitope against which an antibody may be made, or which can be identified by some other agent, yet is short enough such that it does not interfere with the activity of the PRO polypeptide of interest. The tag polypeptide preferably is also fairly unique so that the antibody does not substantially cross-react with other epitopes. Suitable tag polypeptides generally have at least six amino acid residues and usually between about 8 to about 50 amino acid residues (preferably, between about 10 to about 20 residues).

"Isolated, when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO polypeptide natural environment will not be present. Ordinarily, however, isolated polypeptide will be prepared by at least one purification step.

An "isolated" PRO polypeptide-encoding nucleic acid is a nucleic acid molecule that is identified and separated from at least one contaminant nucleic acid molecule with which it is ordinarily associated in the natural source of the PRO polypeptide nucleic acid. An isolated PRO polypeptide nucleic acid molecule is other than in the form or setting in which it is found in nature. Isolated PRO polypeptide nucleic acid molecules therefore are distinguished from the specific PRO polypeptide nucleic acid molecule as it exists in natural cells. However, an isolated PRO polypeptide nucleic acid molecule includes PRO polypeptide nucleic acid molecules contained in cells that ordinarily express the PRO polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used ' in accordance with conventional practice.

The term "antibody" is used in the broadest sense and specifically covers single anti-PRO polypeptide monoclonal antibodies (including agonist, antagonist, and neutralizing antibodies) and anti-PRO polypeptide antibody compositions with polyepitopic specificity. The term "monoclonal antibody" as used herein refers to an antibody obtained from a population of substantially homogeneous antibodies, *i.e*., the individual antibodies comprising the population are identical except for possible naturally-occurring mutations that may be present in minor amounts.

"Active" or "activity" for the purposes herein refers to form(s) of PRO polypeptide which retain the biologic and/or immunologic activities of the specific native or naturally-occurring PRO polypeptide.

"Treatment" or "treating" refers to both therapeutic treatment and prophylactic or preventative measures. Those in need of treatment include those already with the disorder as well as those prone to have the disorder of those in which the disorder is to be prevented.

"Mammal" for purposes of treatment refers to any animal classified as a mammal, including humans, domestic and farm animals, and zoo, sports, or pet animals, such as sheep, dogs, horses, cats, cows, and the like. Preferably, the mammal herein is a human.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, polyethylene glycol (PEG), and PLURONICS^{™}.

The term "agonist" is used to refer to peptide and non-peptide analogs of the native PRO polypeptides (where native PRO polypeptide refers to pro-PRO polypeptide, pre-PRO polypeptide, prepro-PRO polypeptide, or mature PRO polypeptide) of the present invention and to antibodies specifically binding such native PRO polypeptides, provided that they retain at least one biological activity of a native PRO polypeptide. Preferably, the agonists of the present invention retain the qualitative binding recognition properties and receptor activation properties of the native PRO polypeptide.

The term "antagonist" is used to refer to a molecule inhibiting a biological activity of a native PRO polypeptide of the present invention wherein native PRO polypeptide refers to pro-PRO polypeptide, pre-PRO polypeptide, prepro-PRO polypeptide, or mature PRO polypeptide. Preferably, the antagonists herein inhibit the binding of a native PRO polypeptide of the present invention to a binding partner. A PRO polypeptide "antagonist" is a molecule which prevents, or interferes with, a PRO antagonist effector function (e.g. a molecule which prevents or interferes with binding and/or activation of a PRO polypeptide receptor by PRO polypeptide). Such molecules can be screened for their ability to competitively inhibit PRO polypeptide receptor activation by monitoring binding of native PRO polypeptide in the presence and absence of the test antagonist molecule, for example. An antagonist of the invention also encompasses an antisense polynucleotide against the PRO polypeptide gene, which antisense polynucleotide blocks transcription or translation of the PRO polypeptide gene, thereby inhibiting its expression and biological activity.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel *et al.,* Current Protocols in Molecular Biology, Wiley Interscience Publishers, (1995).

"Stringent conditions" means (1) employing low ionic strength and high temperature for washing, for example, 0.015 sodium chloride/0.0015 M sodium citrate/0.1 % sodium dodecyl sulfate at 50°C, or (2) employing during hybridization a denaturing agent, such as formamide, for example, 50% (vol/vol) formamide with 0.1 % bovine serum albumin/0.1 % Ficoll/0.1 % polyvinylpyrrolidone/50 nM sodium phosphate buffer at pH 6.5 with 750 mM sodium chloride, 75 mM sodium citrate at 42°C. Another example is use of 50% formamide, 5 x SSC (0.75 M NaCl, 0.075 M sodium citrate), 50 mM sodium phosphate (pH 6/8), 0.1 % sodium pyrophosphate, 5 x Denhardt's solution, sonicated salmon sperm DNA (50 *µ*g/ml), 0.1 % SDS, and 10% dextran sulfate at 42°C, with washes at 42°C in 0.2 x SSC and 0.1 % SDS. Yet another example is hybridization using a buffer of 10% dextran sulfate, 2 x SSC (sodium chloride/sodium citrate) and 50% formamide at 55°.C, followed by a high-stringency wash consisting of 0.1 x SSC containing EDTA at 55°C.

"Moderately stringent conditions" are described in Sambrook *et al., supra,* and include the use of a washing solution and hybridization conditions (*e.g*., temperature, ionic strength, and %SDS) less stringent than described above. An example of moderately stringent conditions is a condition such as overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/mL denatured sheared salmon sperm DNA, followed by washing the filters in 1 x SSC at about 37-50°C. The skilled artisan will recognize how to adjust the temperature, ionic strength, *etc.,* as necessary to accommodate factors such as probe length and the like.

"Southern analysis* or "Southern blotting" is a method by which the presence of DNA sequences in a restriction endonuclease digest of DNA or a DNA-containing composition is confirmed by hybridization to a known, labeled oligonucleotide or DNA fragment. Southern analysis typically involves electrophoretic separation of DNA digests on agarose gels, denaturation of the DNA after electrophoretic separation, and transfer of the DNA to nitrocellulose, nylon, or another suitable membrane support for analysis with a radiolabeled, biotinylated, or enzyme-labeled probe as described in sections 9.37-9.52 of Sambrook *et al.,* Molecular Cloning: A Laboratory Manual (New York: Cold Spring Harbor Laboratory Press, 1989).

"Northern analysis" or "Northern blotting" is a method used to identify RNA sequences that hybridize to a known probe such as an oligonucleotide, DNA fragment, cDNA or fragment thereof, or RNA fragment. The probe is labeled with a radioisotope such as ³²P, or by biotinylation, or with an enzyme. The RNA to be analyzed is usually electrophoretically separated on an agarose or polyacrylamide gel, transferred to nitrocellulose, nylon, or other suitable membrane, and hybridized with the probe, using standard techniques well known in the art such as those described in sections 7.39-7.52 of Sambrook *et al., supra.*

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Chronic" administration refers to administration of the agent(s) in a continuous mode as opposed to an acute mode, so as to maintain the initial therapeutic effect (activity) for an extended period of time. ''intermittent" administration is treatment that is not consecutively done without interruption, but rather is cyclic in nature.

Administration "in combination with" one or more further therapeutic agents includes simultaneous (concurrent) and consecutive administration in any order.

The term "expression vector" is used to define a vector, in which a nucleic acid encoding a PRO polypeptide herein is operably linked to control sequences capable of affecting its expression is a suitable host cells. Vectors ordinarily carry a replication site (although this is not necessary where chromosomal integration will occur). Expression vectors also include marker sequences which are capable of providing phenotypic selection in transformed cells. For example, E. coli is typically transformed using pBR322, a plasmid derived from an *E*. *coli* species (Bolivar, *et al.,* Gene 2: 95 [1977]). pBR322 contains genes for ampicillin and tetracycline resistance and thus provides easy means for identifying transformed cells, whether for purposes of cloning or expression. Expression vectors also optimally will contain sequences which are useful for the control of transcription and translation, e.g., promoters and Shine-Dalgarno sequences (for prokaryotes) or promoters and enhancers (for mammalian cells). The promoters may be, but need not be, inducible; even powerful constitutive promoters such as the CMV promoter for mammalian hosts have been found to produce the LHR without host cell toxicity. While it is conceivable that expression vectors need not contain any expression control, replicative sequences or selection genes, their absence may hamper the identification of hybrid transformants and the achievement of high level hybrid immunoglobulin expression.

The term "lipopolysaccharide" or "LPS" is used herein as a synonym of "endotoxin." Lipopolysaccharides (LPS) are characteristic components of the outer membrane of Gram-negative bacteria, e.g., *Escherichia coli.* They consist of a polysaccharide part and a fat called lipid A. The polysaccharide, which varies from one bacterial species to another, is made up of the O-specific chain (built from repeating units of three to eight sugars) and the two-part core. Lipid A virtually always includes two glucosamine sugars modified by phosphate and a variable number of fatty acids. For further information see, for example, Rietschel and Brade, Scientific American August 1992, 54-61.

The term "septic shock" is used herein in the broadest sense, including all definitions disclosed in Bone, Ann. Intern Med. 114, 332-333 (1991). Specifically, septic shock starts with a systemic response to infection, a syndrome called sepsis. When this syndrome results in hypotension and organ dysfunction, it is called septic shock. Septic shock may be initiated by gram-positive organisms and fungi, as well as endotoxin-containing Gram-negative organisms. Accordingly, the present defmition is not limited to "endotoxin shock."

The phrases "gene amplification" and "gene duplication" are used interchangeably and refer to a process by which multiple copies of a gene or gene fragment are formed in a particular cell or cell line. The duplicated region (a stretch of amplified DNA) is often referred to as "amplicon". Usually, the amount of the messenger RNA (mRNA) produced, i.e., the level of gene expression, also increases in the proportion of the number of copies made of the particular gene expressed.

"Tumor", as used herein, refers to all neoplastic cell growth and proliferation, whether malignant or bettign, and all pre-cancerous and cancerous cells and tissues. The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include breast cancer, prostate cancer, colon cancer, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colorectal cancer, endometrial carcinoma, salivary gland carcinoma, kidney cancer, vulval cancer, thyroid cancer, hepatic carcinoma and various types of head and neck cancer.

The term "cytotoxic agent" as used herein refers to a substance that inhibits or prevents the function of cells and/or causes destruction of cells. The term is intended to include radioactive isotopes (e.g. 1131, I125, Y90 and Re186), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, e.g. paclitaxel (Taxol, Bristol-Myers Squibb Oncology, Princeton, NJ), and doxetaxel (Taxotere", Rhone-Poulenc Rorer, Antony, France), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either in vitro or in vivo. Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, onoogens, and antineoplastic drugs "by Murakami et al. (WB Saunders: Philadelphia, 1995), especially p.13.

"Doxorubicin" is an athracycline antibiotic.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; and the like. As used herein, the term cytokine includes proteins from natural sources or from recombinant cell culture and biologically active equivalents of the native sequence cytokines.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat et al., NIH Publ. No.91-3242, Vol. I, pages 647-669 (1991)). The constant domains are not involved directly in binding an antibody to an antigen, but exhibit various effector functions, such as participation of the antibody in antibody-dependent cellular toxicity.

"Antibody fragments comprise a portion of an intact antibody, preferably the antigen binding or variable region of the intact antibody. Examples of antibody fragments include Fab, Fab', F(ab')2, and Fv fragments; diabodies; linear antibodies (Zapata et al. , Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')2 fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isorypes), e.g., IgG1, IgG2, IgG3, IgG4, IgA, and IgA2.

''Single-chain Fv" or "sFv" antibody fragments comprise the VH and VL domains of antibody, wherein these domains are present in a single polypeptide chain. Preferably, the Fv polypeptide further comprises a polypeptide linker between the VH and VL domains which enables the sFv to form the desired structure for antigen binding. For a review of sFv see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH - VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95 % by weight of antibody as determined by the Lowry method, and most preferably more than 99 % by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to the antibody so as to generate a "labelled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes.

### II. Compositions and Methods of the Invention

### Full-length PRO181 Polypeptides

Applicants have identified and isolated nucleotide sequences encoding polypeptides referred to in the present application as PRO181. In particular, Applicants have identified and isolated cDNA encoding a PRO181 polypeptide, as disclosed in further detail in the Examples below. Using BLAST and FastA sequence alignment computer programs, Applicants found that the PRO181 polypeptide has significant similarity to the cornichon protein. Accordingly, it is presently believed that PRO181 polypeptide disclosed in the present application is a cornichon homolog.

### PRO Polypeptide Variants

In addition to the full-length native sequence PRO polypeptides described herein, it is contemplated that PRO polypeptide variants can be prepared. PRO polypeptide variants can be prepared by introducing appropriate nucleotide changes into the PRO polypeptide DNA, or by synthesis of the desired PRO polypeptide. Those skilled in the art will appreciate that amino acid changes may alter post-translational processes of the PRO polypeptides, such as changing the number or position of glycosylation sites or altering the membrane anchoring characteristics.

Variations in the native full-length sequence PRO polypeptides or in various domains of the PRO polypeptides described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO polypeptide that results in a change in the amino acid sequence of the PRO polypeptide as compared with the native sequence PRO polypeptide. Optionally the variation is by substitution of at least one amino acid with any other amino acid in one or more of the domains of the PRO polypeptide. Guidance in determining which amino acid residue may be inserted, substituted or deleted without adversely affecting the desired activity may be found by comparing the sequence of the PRO polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of 1 to 5 amino acids. The variation allowed may be determined by systematically making insertions, deletions or substitutions of amino acids in the sequence and testing the resulting variants for activity in the *in vitro* assay described in the Examples below.

In particular embodiments, conservative substitutions of interest are shown in Table 1 under the heading of preferred substitutions. If such substitutions result in a change in biological activity, then more substantial changes, denominated exemplary substitutions in Table 1, or as further described below in reference to amino acid classes, are introduced and the products screened.

**Table 1**

| Original Residue | Exemplary Substitutions | Preferred Substitutions |
|---|---|---|
| Ala (A) | val; leu; ile | val |
| Arg (R) | lys; gln; asn | lys |
| Asn (N) | gin; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; | |
| | met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the desired PRO polypeptide variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### Modifications of PRO Polypeptides

Covalent modifications of PRO polypeptides are included within the scope of this invention. One type of covalent modification includes reacting targeted amino acid residues of the PRO polypeptide with an organic derivatizing agent that is capable of reacting with selected side chains or the N- or C- terminal residues of the PRO polypeptide. Derivatization with bifunctional agents is useful, for instance, for crosslinking a PRO polypeptide to a water-insoluble support matrix or surface for use in the method for purifying anti-PRO polypeptide antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)-dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptides included within the scope of this invention comprises altering the native glycosylation pattern of the polypeptide. "Altering the native glycosylation pattern" is intended for purposes herein to mean deleting one or more carbohydrate moieties found in a native sequence PRO polypeptide, and/or adding one or more glycosylation sites that are not present in the native sequence PRO polypeptide, and/or alteration of the ratio and/or composition of the sugar residues attached to the glycosylation site(s).

Addition of glycosylation sites to the PRO polypeptide may be accomplished by altering the amino acid sequence. The alteration may be made, for example, by the addition of, or substitution by, one or more serine or threonine residues to the native sequence PRO polypeptide (for O-linked glycosylation sites). The PRO polypeptide amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO polypeptides of the invention comprises linking the PRO polypeptide to one of a variety of nonproteinaceous polymers, *e.g*., polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

The PRO polypeptides of the present invention may also be modified in a way to form a chimeric molecule comprising a PRO polypeptide fused to another, heterologous polypeptide or amino acid sequence. In one embodiment, such a chimeric molecule comprises a fusion of the PRO polypeptide with a tag polypeptide which provides an epitope to which an anti-tag antibody can selectively bind. The epitope tag is generally placed at the amino- or carboxyl- terminus of the PRO polypeptide. The presence of such epitope-tagged forms of the PRO polypeptide can be detected using an antibody against the tag polypeptide. Also, provision of the epitope tag enables the PRO polypeptide to be readily purified by affinity purification using an anti-tag antibody or another type of affinity matrix that binds to the epitope tag. In an alternative embodiment, the chimeric molecule may comprise a fusion of the PRO polypeptide with an immunoglobulin or a particular region of an immunoglobulin. For a bivalent form of the chimeric molecule, such a fusion could be to the Fc region of an IgG molecule.

Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

### Preparation of PRO Polypeptides

The description below relates primarily to production of PRO polypeptides by culturing cells transformed or transfected with a vector containing the desired PRO polypeptide nucleic acid. It is, of course, contemplated that alternative methods, which are well known in the art, may be employed to prepare the PRO polypeptide. For instance, the PRO polypeptide sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the desired PRO polypeptide may be chemically synthesized separately and combined using chemical or enzymatic methods to produce the full-length PRO polypeptide.

### A. Isolation of DNA Encoding PRO Polypeptides

DNA encoding PRO polypeptides may be obtained from a cDNA library prepared from tissue believed to possess the desired PRO polypeptide mRNA and to express it at a detectable level. Accordingly, human PRO polypeptide DNA can be conveniently obtained from a cDNA library prepared from human tissue, such as described in the Examples. The PRO polypeptide-encoding gene may also be obtained from a genomic library or by oligonucleotide synthesis.

Libraries can be screened with probes (such as antibodies to the desired PRO polypeptide or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York- Cold Spring Harbor Laboratory Press, 1989). An alternative means to isolate the gene encoding the desired PRO polypeptide is to use PCR methodology [Sambrook et al., supra; Dieffenbach et al., PCR Primer:A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined through sequence alignment using computer software programs such as BLAST, ALIGN, DNAstar, and INHERIT which employ various algorithms to measure homology.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### B. Selection and Transformation of Host Cells

Host cells are transfected or transformed with expression or cloning vectors described herein for PRO polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach. M. Butler, ed. (IRL Press, 1991) and Sambrook et al., supra.

Methods of transfection are known to the ordinarily skilled artisan, for example, CaPO₄ and electroporation. Depending on the host cell used, transformation is performed using standard techniques appropriate to such cells. The calcium treatment employing calcium chloride, as described in Sambrook et al., supra, or electroporation is generally used for prokaryotes or other cells that contain substantial cell-wall barriers. Infection with *Agrobactitium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transformations have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, *e.g*., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E*. *coli* strains are publicly available, such as *E*. *coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia, e.g., E. coli, Enterobacter, Erwinia, Klebsiella, Proteus, Salmonella, e.g., Salmonella typhimurium, Serratia, e.g., Serratia marcescans,* and *Shigella,* as well as *Bacilli* such as B. *subtilis* and *B. licheniformis (e.g., B. licheniformis* 41P disclosed in DD 266,710 published 12 April 1989), *Pseudomonas* such as *P. aeruginosa,* and *Streptomyces.* Various *E. coli* strains are publicly available, such as *E. coli* K12 strain MM294 (ATCC 31,446); *E*. *coli* X1776 (ATCC 31,537); *E. coli* strain W3110 (ATCC 27,325); and K5 772 (ATCC 53,635). These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA ; E. coli* W3110 strain 9E4, which has the complete genotype *tonA ptr3; E. coli* W3110 strain 27C7 (ATCC 55,244), which has the complete genotype *tonA pir3 phoA E15 (argF-lac)169 degP ompT kan*^{*r*}*; E. coli* W3110 strain 37D6, which has the complete genotype *tonA ptr3 phoA E15 (argF-lac)169 degP ompT rbs7 ilvG kan*^{*r*}*; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, *e.g.,* PCR or other nucleic acid polymerase reactions, are suitable.

In addition to prokaryotes, eukaryotic microbes such as filamentous fungi or yeast are suitable cloning or expression hosts for PRO polypeptide-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer *et al.,* Bio/Technology, 9: 968-975 (1991)) such as, *e.g. , K. lactis* (MW98-8C, CBS683, CBS4574; Louvencourt *et al.,* J. Bacteriol., 737 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg *et al.,* Bio/Technology, 8: 135 (1990)), *K* . *thermotolerans,* and *K. marxianus; yarrowia* (EP 402,226); *Pichia pastoris* (EP 183,070; Sreekrishna *et al.,* J. Basic Microbiol., 28: 265-278 [1988]); *Candida, Trichoderma reesia* (EP 244,234); *Neurospora crassa* (Case *et al.,* Proc. Natl. Acad. Sci. USA, 76: 5259-5263 [1979]); *Schwanniomyces* such as *Schwanniomyces occidentalis* (EP 394,538 published 31 October 1990); and filamentous fungi such as, *e.g., Neurospora, Penicillium, Tolypocladium* (WO 91/00357 published 10 January 1991), and *Aspergillus* hosts such as *A. nidulans* (Ballance *et al.,* Biochem. Biophys. Res. Commun., 112: 284-289 [1983]; Tilburn *et al.,* Gene, 26: 205-221 [1983]; Yelton *et al.,* Proc. Natl. Acad. Sci. USA, 81: 1470-1474 [1984]) *and A. niger (Kelly* and Hynes, EMBO J., 4: 475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO polypeptides are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### C. Selection and Use of a Replicable Vector

The nucleic acid (e.g., cDNA or genomic DNA) encoding a desired PRO polypeptide may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO polypeptide of interest may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO polypeptide DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C*. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PRO polypeptide nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7: 141 (1979); Tschemper et al., Gene, 10: 157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO polypeptide nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl, Acad. Sci. USA, 80:21-25 (1983)]. Promoters for use in bacterial systems also will contain a Shine-Dalgarno (S.D.) sequence operably linked to the DNA encoding the desired PRO polypeptide.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO polypeptide transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g*., the actin promoter or an immunoglobulin promoter, and from heat-shock promoters, provided such promoters are compatible with the host cell systems.

Transcription of a DNA encoding the desired PRO polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO polypeptide coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO polypeptides.

Still other methods, vectors, and host cells suitable for adaptation to the synthesis of PRO polypeptides in recombinant vertebrate cell culture are described in Gething et al., Nature, 293:620-625 (1981); Mantei et al., Nature, 281:40-46 (1979); EP 117,060; and EP 117,058.

### D. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to a PRO polypeptide DNA and encoding a specific antibody epitope.

### E. Purification of Polypeptide

Forms of PRO polypeptides may be recovered from culture medium or from host cell lysates. If membrane-bound, it can be released from the membrane using a suitable detergent solution (*e.g*. Triton-X 100) or by enzymatic cleavage. Cells employed in expression of PRO polypeptides can be disrupted by various physical or chemical means, such as freeze-thaw cycling, sonication, mechanical disruption, or cell lysing agents.

It may be desired to purify PRO polypeptides from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO polypeptide. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO polypeptide produced.

### Uses for PRO Polypeptides

Nucleotide sequences (or their complement) encoding the PRO polypeptides of the present invention have various applications in the art of molecular biology, including uses as hybridization probes, in chromosome and gene mapping and in the generation of anti-sense RNA and DNA. PRO polypeptide-encoding nucleic acid will also be useful for the preparation of PRO polypeptides by the recombinant techniques described herein.

The full-length native sequence PRO polypeptide-encoding nucleic acid or portions thereof, may be used as hybridization probes for a cDNA library to isolate the full-length PRO polypeptide gene or to isolate still other genes (for instance, those encoding naturally-occurring variants of the PRO polypeptide or PRO polypeptides from other species) which have a desired sequence identity to the PRO polypeptide nucleic acid sequences. Optionally, the length of the probes will be about 20 to about 50 bases. The hybridization probes may be derived from the nucleotide sequence of any of the DNA molecules disclosed herein or from genomic sequences including promoters, enhancer elements and introns of native sequence PRO polypeptide encoding DNA. By way of example, a screening method will comprise isolating the coding region of the PRO polypeptide gene using the known DNA sequence to synthesize a selected probe of about 40 bases. Hybridization probes may be labeled by a variety of labels, including radionucleotides such as ³²P or ³⁵S, or enzymatic labels such as alkaline phosphatase coupled to the probe via avidin/biotin coupling systems. Labeled probes having a sequence complementary to that of the specific PRO polypeptide gene of the present invention can be used to screen libraries of human cDNA, genomic DNA or mRNA to determine which members of such libraries the probe hybridizes to. Hybridization techniques are described in further detail in the Examples below.

The ESTs disclosed in the present application may similarly be employed as probes, using the methods disclosed herein.

The probes may also be employed in PCR techniques to generate a pool of sequences for identification of closely related PRO polypeptide sequences.

Nucleotide sequences encoding a PRO polypeptide can also be used to construct hybridization probes for mapping the gene which encodes that PRO polypeptide and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

When the coding sequence for the PRO polypeptide encodes a protein which binds to another protein, the PRO polypeptide can be used in assays to identify its ligands. Similarly, inhibitors of the receptor/ligand binding interaction can be identified. Proteins involved in such binding interactions can also be used to screen for peptide or small molecule inhibitors or agonists of the binding interaction. Screening assays can be designed to find lead compounds that mimic the biological activity of a native PRO polypeptide or a ligand for the PRO polypeptide. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode a PRO polypeptide or its modified forms can also be used to generate either transgenic animals or °knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. In one embodiment, cDNA encoding a PRO polypeptide of interest can be used to clone genomic DNA encoding the PRO polypeptide in accordance with established techniques and the genomic sequences used to generate transgenic animals that contain cells which express DNA encoding the PRO polypeptide. Methods for generating transgenic animals, particularly animals such as mice or rats, have become conventional in the art and are described, for example, in U.S. Patent Nos. 4,736,866 and 4,870,009. Typically, particular cells would be targeted for PRO polypeptide transgene incorporation with tissue-specific enhancers. Transgenic animals that include a copy of a transgene encoding a PRO polypeptide introduced into the germ line of the animal at an embryonic stage can be used to examine the effect of increased expression of DNA encoding the PRO polypeptide. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Alternatively, non-human homologues of PRO polypeptides can be used to construct a PRO polypeptide "knock out" animal which has a defective or altered gene encoding the PRO polypeptide of interest as a result of homologous recombination between the endogenous gene encoding the PRO polypeptide and altered genomic DNA encoding the PRO polypeptide introduced into an embryonic cell of the animal. For example, cDNA encoding a PRO polypeptide can be used to clone genomic DNA encoding the PRO polypeptide in accordance with established techniques. A portion of the genomic DNA encoding a PRO polypeptide can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in *Teratocarcinomas and Embryonic Stem Cells: A Practical Approach,* E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the PRO polypeptide.

When *in vivo* administration of a PRO polypeptide is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

Where sustained-release administration of a PRO polypeptide is desired in a formulation with release characteristics suitable for the treatment of any disease or disorder requiring administration of the PRO polypeptide, microencapsulation of the PRO polypeptide is contemplated. Microencapsulation of recombinant proteins for sustained release has been successfully performed with human growth hormone (rhGH), interferon-(rhIFN-), interleukin-2, and MN rgp120. Johnson *et al.,* Nat. Med., 2: 795-799 (1996); Yasuda, Biomed. Ther., 27: 1221-1223 (1993); Hora *et al.,* Bio/Technology, 8: 755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439-462; WO 97/03692, WO 96/40072, WO 96/07399; and U.S Pat. No. 5,654,010.

The sustained-release formulations of these proteins were developed using poly-lactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. Lewis, "Controlled release of bioactive agents from lactide/glycolide polymer," in: M. Chasin and R. Langer (Eds.), Biodegradable Polymers as Drug Delivery Systems (Marcel Dekker: New York, 1990), pp. 1-41.

For example, for a formulation that can provide a dosing of approximately 80 g/kg/day in mammals with a maximum body weight of 85 kg, the largest dosing would be approximately 6.8 mg of the PRO polypeptide per day. In order to achieve this dosing level, a sustained- release formulation which contains a maximum possible protein loading (15-20% w/w PRO polypeptide) with the lowest possible initial burst (< 20%) is necessary. A continuous (zero-order) release of the PRO polypeptide from microparticles for 1-2 weeks is also desirable. In addition, the encapsulated protein to be released should maintain its integrity and stability over the desired release period.

PRO181 polypeptides of the present invention which possess biological activity related to that of the cornichon protein may be employed both *in vivo* for therapeutic purposes and *in vitro.* Those of ordinary skill in the art will well know how to employ the PRO181 polypeptides of the present invention for such purposes.

The compounds of the present invention can be formulated according to known methods to prepare pharmaceutically useful compositions, whereby the PRO polypeptide hereof is combined in admixture with a pharmaceutically acceptable carrier vehicle. Suitable carrier vehicles and their formulation, inclusive of other human proteins, e.g., human serum albumin, are described, for example, in *Remington's Pharmaceutical Sciences,* 16th ed., 1980, Mack Publishing Co., edited by Oslo *et al.*

Dosages and desired drug concentrations of pharmaceutical compositions of the present invention may vary depending on the particular use envisioned. For example, in the treatment of deep vein thrombosis or peripheral vascular disease, "bolus" doses, will typically be preferred with subsequent administrations being given to maintain an approximately constant blood level, preferably on the order of about 3 µg/ml.

However, for use in connection with emergency medical care facilities where infusion capability is generally not available and due to the generally critical nature of the underlying disease (e.g., embofism, infarct), it will generally be desirable to provide somewhat larger initial doses, such as an intravenous bolus.

### Anti-PRO Polypeptide Antibodies

The present invention further provides anti-PRO polypeptide antibodies. Exemplary antibodies include polyclonal, monoclonal, humanized, bispecific, and heteroconjugate antibodies.

### A. Polyclonal Antibodies

The anti-PRO polypeptide antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### B. Monoclonal Antibodies

The anti-PRO polypeptide antibodies may, alternatively, be monoclonal antibodies. Monoclonal antibodies may be prepared using hybridoma methods, such as those described by Kohler and Milstein, *Nature,* 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide of interest or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Rockville, Maryland. Human myeloma and mouse-human heteromyeloma cell lines also have been described for the production of human monoclonal antibodies [Kozbor, *J*. *Immunol.,* 133:3001 (1984); Brodeur *et al., Monoclonal Antibody Production Techniques and Applications,* Marcel Dekker, Inc., New York, (1987) pp. 51-63].

The culture medium in which the hybridoma cells are cultured can then be assayed for the presence of monoclonal antibodies directed against the PRO polypeptide of interest. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, *Anal. Biochem.,* 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods, are also suitable for preparing monovalent antibodies. Digestion of antibodies to produce fragments thereof, particularly, Fab fragments, can be accomplished using routine techniques known in the art.

### C. Humanized Antibodies

The anti-PRO polypeptide antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones *et al., Nature,* 321: 522-525 (1986); Riechmann *et al., Nature,* 332:323-329 (1988); and Presta, *Curr. Op. Struct. Biol. ,* 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones *et al.. Nature,* 321: 522-525 (1986); Riechmann *et al., Nature,* 332:323-327 (1988); Verhoeyen *et al., Science,* 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, *J. Mol. Biol.,* 227:381 (1991); Marks *et al.. J. Mol. Biol.,* 222:581 (1991)]. The techniques of Cole *et al.* and Boerner *et al.* are also available for the preparation of human monoclonal antibodies (Cole *et al., Monoclonal Antibodies and Cancer Therapy,* Alan R. Liss, p. 77 (1985) and Boemer *et al., J. Immunol.,* 147(1):86-95 (1991)].

### D. Bispecific Antibodies

Bispecific antibodies are monoclonal, preferably human or humanized, antibodies that have binding specificities for at least two different antigens. In the present case, one of the binding specificities is for the PRO polypeptide, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, *Nature,* 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker *et al., EMBO J.,* 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh *et al., Methods in Enzymology,* 121:210 (1986).

### E. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyritnidate and those disclosed, for example, in U.S. Patent No. 4,676,980.

### Uses for Anti-PRO Polypeptide Antibodies

The anti-PRO polypeptide antibodies of the invention have various utilities. For example, anti-PRO polypeptide antibodies may be used in diagnostic assays for a PRO polypeptide, *e.g.,* detecting its expression in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³¹P, ³⁵S, or ¹²⁵I. a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter *et al., Nature,* 144:945 (1962); David *et al., Biochemistry,* 13:1014 (1974); Pain *et al,. J. Immunol. Meth.,* 40:219 (1981); and Nygren. *J. Histochem. and Cytochem"* 30:407 (1982).

Anti-PRO polypeptide antibodies also are useful for the affinity purification of PRO polypeptide from recombinant cell culture or natural sources. In this process, the antibodies against the PRO polypeptide are immobilized on a suitable support, such a Sephadex resin or filter paper, using methods well known in the art. The immobilized antibody then is contacted with a sample containing the PRO polypeptide to be purified, and thereafter the support is washed with a suitable solvent that will remove substantially all the material in the sample except the PRO polypeptide, which is bound to the immobilized antibody. Finally, the support is washed with another suitable solvent that will release the PRO polypeptide from the antibody.

The following examples are offered for illustrative purposes only, and are not intended to limit the scope of the present invention in any way.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Rockville, Maryland.

### EXAMPLE 1: Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (e.g., Dayhoff, GenBank), and proprietary databases (e.g. LIFESEQ^{™} Incyte Pharmaceuticals, Palo Alto, CA). The search was performed using the computer program BLAST or BLAST2 (Altschul and Gish, Methods in Enzymology 266: 460-480 (1996)) as a comparison of the ECD protein sequences to a 6 frame translation of the EST sequences. Those comparisons with a Blast score of 70 (or in some cases 90) or greater that did not encode known proteins were clustered and assembled into consensus DNA sequences with the program "phrap" (Phil Green, University of Washington, Seattle, WA; (http://bozeman. mbt.washington.edu/phrap.dors/phrap.html).

Using this extracellular domain homology screen, consensus DNA sequences were assembled relative to the other identified EST sequences using phrap. In addition, the consensus DNA sequences obtained were often (but not always) extended using repeated cycles of BLAST and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward (.f) and reverse (.r) PCR primers generally range from 20 to 30 nucleotides and are often designed to give a PCR product of about 100-1000 bp in length. The probe (.p) sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemilanased adaptors, cleaved with NotI, sized appropriately by gel electrophoresis, and cloned in a defined orientation into a suitable cloning vector (such as pRKB or pRKD; pRK5B is a precursor of pRKSD that does not contain the SfiI site; see, Holmes et al., Science, 253: 1278-1280 (1991)) in the unique XhoI and NotI sites.

### EXAMPLE 2: Isolation of cDNA clones by Amylase Screening

### 1. Preparation of oligo dT primed cDNA library

mRNA was isolated from a human tissue of interest using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

### 2. Preparation of random primed cDNA library

A secondary cDNA library was generated in order to preferentially represent the 5' ends of the primary cDNA clones. Sp6 RNA was generated from the primary library (described above), and this RNA was used to generate a random primed cDNA library in the vector pSST-AMY.O using reagents and protocols from Life Technologies (Super Script Plasmid System, referenced above). In this procedure the double stranded cDNA was sized to 504-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

### 3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, e.g. CsCl-gradient. The purified DNA was then carried on to the yeast protocols below.

The yeast methods were divided into three categories: (1) Transformation of yeast with the plasmid/cDNA combined vector; (2) Detection and isolation of yeast clones secreting amylase; and (3) PCR amplification of the insert directly from the yeast colony and purification of the DNA for sequencing and further analysis.

The yeast strain used was HD56-5A (ATCC-90785). This strain has the following genotype: MAT alpha, ura3-52, leu2-3, leu2-112, his3-11, his3-15, MAL⁺, SUC⁺, GAL⁺. Preferably, yeast mutants can be employed that have deficient post-translational pathways. Such mutants may have translocation deficient alleles in *sec*71*, sec72, sec62,* with truncated *sec*71 being most preferred. Alternatively, antagonists (including antisense nucleotides and/or ligands) which interfere with the normal operation of these genes, other proteins implicated in this post translation pathway (e.g., SEC61p, SEC72p, SEC62p, SEC63p, TDJ1p or SSA1p-4p) or the complex formation of these proteins may also be preferably employed in combination with the amylase-expressing yeast.

Transformation was performed based on the protocol outlined by Gietz et al., Nucl. Acid. Res., 20:1425 (1992). Transformed cells were then inoculated from agar into YEPD complex media broth (100 ml) and grown overnight at 30°C. The YEPD broth was prepared as described in Kaiser et al., Methods in Yeast Genetics, Cold Spring Harbor Press, Cold Spring Harbor, NY, p. 207 (1994). The overnight culture was then diluted to about 2 x 10⁶ cells/ml (approx. OD₆₀₀=0.1) into fresh YEPD broth (500 ml) and regrown to 1 x 10⁷ cells/ml (approx. OD₆₀₀=0.4-0.5).

The cells were then harvested and prepared for transformation by transfer into GS3 rotor bottles in a Sorval GS3 rotor at 5,000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCI, 1 mM EDTA pH 7.5, 100 mM Li₂OCCH₃), and resuspended into LiAc/TE (2.5 ml).

Transformation took place by mixing the prepared cells (100 *µ*l) with freshly denatured single stranded salmon testes DNA (Lofstrand Labs, Gaithersburg, MD) and transforming DNA (1 *µ*g, vol. < 10 *µ*l) in microfuge tubes. The mixture was mixed briefly by vortexing, then 40% PEG/TE (600 *µ*l, 40% polyethylene glycol-4000, 10 mM Tris-HCl. 1 mM EDTA, 100 mM Li₂OOCCH₃, pH 7.5) was added. This mixture was gently mixed and incubated at 30°C while agitating for 30 minutes. The cells were then heat shocked at 42°C for 15 minutes, and the reaction vessel centrifuged in a microfuge at 12,000 rpm for 5-10 seconds, decanted and resuspended into TE (500 *µ*l, 10 mM Tris-HCl, 1 mM EDTA pH 7.5) followed by recentrifugation. The cells were then diluted into TE (1 ml) and aliquots (200 *µ*l) were spread onto the selective media previously prepared in 150 mm growth plates (VWR).

Alternatively, instead of multiple small reactions, the transformation was performed using a single, large scale reaction, wherein reagent amounts were scaled up accordingly.

The selective media used was a synthetic complete dextrose agar lacking uracil (SCD-Ura) prepared as described in Kaiser et al., Methods in Yeast Genetics, Cold Spring Harbor Press, Cold Spring Harbor, NY, p. 208-210 (1994). Transformants were grown at 30°C for 2-3 days.

The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15% (w/v), and was buffered with potassium phosphate to a pH of 7.0 (50-100 mM final concentration).

The positive colonies were picked and streaked across fresh selective media (onto 150 mm plates) in order to obtain well isolated and identifiable single colonies. Well isolated single colonies positive for amylase secretion were detected by direct incorporation of red starch into buffered SCD-Ura agar. Positive colonies were determined by their ability to break down starch resulting in a clear halo around the positive colony visualized directly.

### 4. Isolation of DNA by PCR Amplification

When a positive colony was isolated, a portion of it was picked by a toothpick and diluted into sterile water (30 *µ*l) in a 96 well plate. At this time, the positive colonies were either frozen and stored for subsequent analysis or immediately amplified. An aliquot of cells (5 *µ*l) was used as a template for the PCR reaction in a 25 *µ*l volume containing: 0.5 *µ*l Klentaq (Clontech, Palo Alto, CA); 4.0 *µ*l 10 mM dNTP's (Perkin Elmer-Cetus); 2.5 *µ*l Kentaq buffer (Clontech); 0.25 *µ*l forward oligo 1; 0.25 *µ*l reverse oligo 2; 12.5 *µ*l distilled water. The sequence of the forward oligonucleotide 1 was:
5'-TGTAAAACGACGGCCAGTTAAATAGACCTGCAATTATTAATCT-3' (SEQ ID NO:4)
The sequence of reverse oligonucleotide 2 was:
5'-CAGGAAACAGCTATGACCACCTGCACACCTGCAAATCCATT-3' (SEQ ID NO:5)

PCR was then performed as follows:

| | | | | |
|---|---|---|---|---|
| a. | | Denature | 92°C, | 5 minutes |
| b. | 3 cycles of: | Denature | 92°C, | 30 seconds |
| | | Anneal | 59°C, | 30 seconds |
| | | Extend | 72°C, | 60 seconds |
| c. | 3 cycles of: | Denature | 92°C. | 30 seconds |
| | | Anneal | 57°C, | 30 seconds |
| | | Extend | 72°C, | 60 seconds |
| d. | 25 cycles of: | Denature | 92°C. | 30 seconds |
| | | Anneal | 55°C, | 30 seconds |
| | | Extend | 72°C, | 60 seconds |
| e. | | Hold | 4°C | |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.

Following the PCR, an aliquot of the reaction (5 *µ*l) was examined by agarose gel electrophoresis in a 1% agarose gel using a Tris-Borate-EDTA (TBE) buffering system as described by Sambrook et al., supra. Clones resulting in a single strong PCR product larger than 400 bp were further analyzed by DNA sequencing after purification with a 96 Qiaquick PCR clean-up column (Qiagen Inc., Chatsworth. CA).

### EXAMPLE 3: Isolation of cDNA Clones Encoding Human PRO181

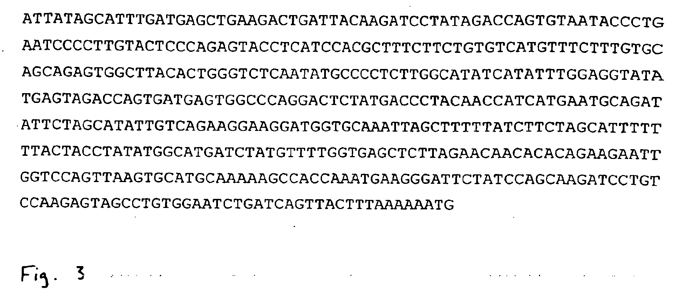

A cDNA sequence isolated in the amylase screen described in Example 2 above was found, by BLAST and FastA sequence alignment, to have sequence homology to a nucleotide sequence encoding the cornichon protein. This cDNA sequence is herein designated DNA 13242 (Figure 3; SEQ ID NO:3). Based on the sequence homology, oligonucleotide probes were generated from the sequence of the DNA13242 molecule and used to screen a human placenta (LIB89) library prepared as described in paragraph 1 of Example 2 above. The cloning vector was pRK5B (pRK5B is a precursor of pRK5D that does not contain the SfiI site; see, Holmes et al., Science, 253:1278-1280 (1991)), and the cDNA size cut was less than 2800 bp.

The oligonucleotide probes employed included:
forward PCR primer 5'-GTGCAGCAGAGTGGCTTACA-3' (SEQ ID NO:6)
reverse PCR primer 5'-ACTGGACCAATTCTTCTGTG-3' (SEQ ID NO:7)
hybridization probe 5'-GATATTCTAGCATATTGTCAGAAGGAAGGATGGTGCAAATTAGCT-3' (SEQ ID NO:8)

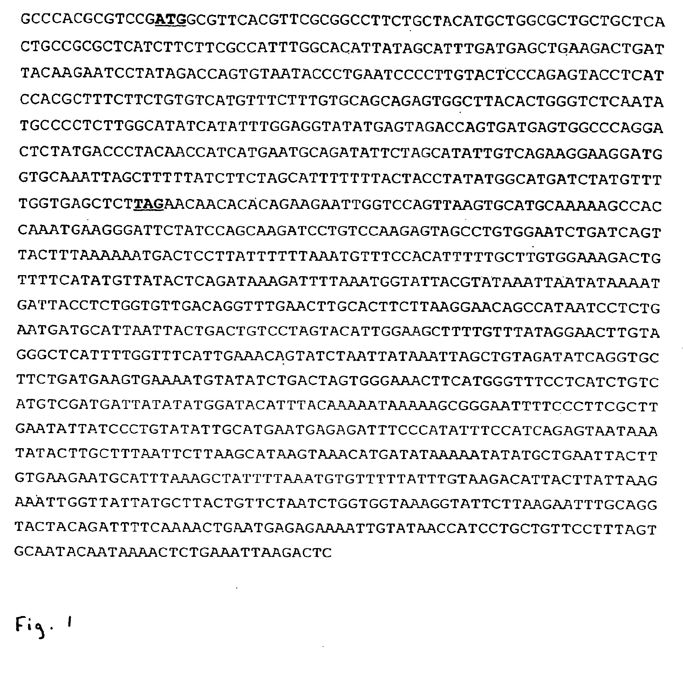

A full length clone was identified that contained a single open reading frame with an apparent translational initiation site at nucleotide positions 14-16 and ending at the stop codon found at nucleotide positions 446-448 (Figure 1; SEQ ID NO:1). The predicted polypeptide precursor is 144 amino acids long, has a calculated molecular weight of approximately 16,699 daltons and an estimated pI of approximately 5.6. Analysis of the full-length PRO181 sequence shown in Figure 2 (SEQ ID NO:2) evidences the presence of the following: a signal peptide from about amino acid 1 to about amino acid 20, a putative type II transmembrane domain from about amino acid 11 to about amino acid 31 and other transmembrane domains from about amino acid 57 to about amino acid 77 and from about amino acid 123 to about amino acid 143. Clone UNQ155 (DNA23330-1390) has been deposited with ATCC on April 14, 1998 and is assigned ATCC deposit no. 209775.

Analysis of the amino acid sequence of the full-length PRO181 polypeptide suggests that it possesses significant sequence similarity to the cornichon protein, thereby indicating that PRO181 may be a novel cornichon homolog. More specifically, an analysis of the Dayhoff database (version 35.45 SwissProt 35) evidenced significant homology between the PRO181 amino acid sequence and the following Dayhoff sequences, AFO22811_1, CET09E8_3, S64058, YGF4_YEAST, YB60_YEAST, EBU89455_1, SIU36383 3 and PH1371.

### EXAMPLE 4: Use of PRO Polypeptide-Encoding Nucleic Acid as Hybridization Probes

The following method describes use of a nucleotide sequence encoding a PRO polypeptide as a hybridization probe.

DNA comprising the coding sequence of of a PRO polypeptide of interest as disclosed herein may be employed as a probe or used as a basis from which to prepare probes to screen for homologous DNAs (such as those encoding naturally-occurring variants of the PRO polypeptide) in human tissue cDNA libraries or human tissue genomic libraries.

Hybridization and washing of filters containing either library DNAs is performed under the following high stringency conditions. Hybridization of radiolabeted PRO polypeptide-encoding nucleic acid-derived probe to the filters is performed in a solution of 50% fotinamide, 5x SSC, 0. 1 % SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1 % SDS at 42°C.

DNAs having a desired sequence identity with the DNA encoding full-length native sequence PRO polypeptide can then be identified using standard techniques known in the art.

### EXAMPLE 5: Expression of PRO Polypeptides in E. coli

This example illustrates preparation of an unglycosylated form of a desired PRO polypeptide by recombinant expression in *E. coli.*

The DNA sequence encoding the desired PRO polypeptide is initially amplified using selected PCR primers. The primers should contain restriction enzyme sites which correspond to the restriction enzyme sites on the selected expression vector. A variety of expression vectors may be employed. An example of a suitable vector is pBR322 (derived from *E*. *coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the specific PRO polypeptide coding region, lambda transcriptional terminator, and an argU gene.

The ligation mixture is then used to transform a selected *E*. *coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO polypeptide can then be purified using a metal chelating column under conditions that allow tight binding of the protein.

PRO181 was expressed in *E*. *coli* in a poly-His tagged form, using the following procedure. The DNA encoding the PRO polypeptide was initially amplified using selected PCR primers. The primers contained restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences were then ligated into an expression vector, which was used to transform an *E*. *coli* host based on strain 52 (W3110 fuhA(tonA) Ion galE rpoHts(htpRts) clpP(1acIq). Transformants were first grown in LB containing 50 mg/ml carbenicillin at 30°C with shaking until an O.D.600 of 3-5 was reached. Cultures were then diluted 50-100 fold into CRAP media (prepared by mixing 3.57 g (NH₄)₂SO₄, 0.71 g sodium citrate 2H2O, 1.07 g KCI, 5.36 g Difco yeast extract, 5.36 g Sheffeld hycase SF in 500 mL water, as well as 110 mM MPOS, pH 7.3, 0.55 % (w/v) glucose and 7 mM MgSO₄) and grown for approximately 20-30 hours at 30°C with shaking. Samples were removed to verify expression by SDS-PAGE analysis, and the bulk culture is centrifuged to pellet the cells. Cell pellets were frozen until purification and refolding.

E. coli paste from 0.5 to 1 L fermentations (6-10 g pellets) was resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution was stirred overnight at 4°C. This step results in a denatured protein with all cysteine residues blocked by sulfitolization. The solution was centrifuged at 40,000 rpm in a Beckman Ultracentifuge for 30 min. The supernatant was diluted with 3-5 volumes of metal chelate column buffer (6 M guanidine, 20 mM Tris, pH 7.4) and filtered through 0.22 micron filters to clarify. Depending the clarified extract was loaded onto a 5 ml Qiagen Ni-NTA metal chelate column equilibrated in the metal chelate column buffer. The column was washed with additional buffer containing 50 mM imidazole (Calbiochem, Utrol grade), pH 7.4. The protein was eluted with buffer containing 250 mM imidazole. Fractions containing the desired protein were pooled and stored at 4°C. Protein concentration was estimated by its absorbance at 280 nm using the calculated extinction coefficient based on its amino acid sequence.

The proteins were refolded by diluting sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes were chosen so that the final protein concentration was between 50 to 100 micrograms/ml. The refolding solution was stirred gently at 4°C for 12-36 hours. The refolding reaction was quenched by the addition of TFA to a final concentration of 0.4% (pH of approximately 3). Before further purification of the protein, the solution was filtered through a 0.22 micron filter and acetonitrile was added to 2-10% final concentration. The refolded protein was chromatographed on a Poros R1/H reversed phase column using a mobile buffer of 0.1% TFA with elution with a gradient of acetonitrile from 10 to 80%. Aliquots of fractions with A280 absorbance were analyzed on SDS polyacrylamide gels and fractions containing homogeneous refolded protein were pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO proteins were pooled and the acetonitrile removed using a gentle stream of nitrogen directed at the solution. Proteins were formulated into 20 mM Hepes, pH 6.8 with 0.14 M sodium chloride and 4 % mannitol by dialysis or by gel filtration using G25 Superfine (Pharmacia) resins equilibrated in the formulation buffer and sterile filtered.

### EXAMPLE 6: Expression of PRO Polypeptides in Mammalian Cells

This example illustrates preparation of a glycosylated form of a desired PRO polypeptide by recombinant expression in mammalian cells.

The vector, pRK5 (see EP 307,247, published March 15, 1989), is employed as the expression vector. Optionally, the PRO polypeptide-encoding DNA is ligated into pRK5 with selected restriction enzymes to allow insertion of the PRO polypeptide DNA using ligation methods such as described in Sambrook et al., supra. The resulting vector is called pRK5-PRO polypeptide.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PRO polypeptide DNA is mixed with about 1 µg DNA encoding the VA RNA gene [Thimmappaya et al., Cell, 31:543 (1982)] and dissolved in 500 *µ*l of 1 mM Tris-HCl. 0.1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 *µ*Ci/ml ³⁵S-cysteine and 200 *µ*Ci/ml ³⁵S-methionine. After a 12 hour incubation, the conditioned medium is collected, concentrated on a spin filter, and loaded onto a 15 % SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO polypeptide may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PRO polypeptide DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed PRO polypeptide can then be concentrated and purified by any selected method, such as dialysis and/or column chromatography.

In another embodiment, PRO polypeptides can be expressed in CHO cells. The pRK5-PRO polypeptide can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of PRO polypeptide, the culture medium may be replaced with serum free medium. Preferably, the cultures are incubated for about 6 days, and then the conditioned medium is harvested. The medium containing the expressed PRO polypeptide can then be concentrated and purified by any selected method.

Epitope-tagged PRO polypeptide may also be expressed in host CHO cells. The PRO polypeptide may be subcloned out of the pRK5 vector. The subclone insert can undergo PCR to fuse in frame with a selected epitope tag such as a poly-his tag into a Baculovirus expression vector. The poly-his tagged PRO polypeptide insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO polypeptide can then be concentrated and purified by any selected method, such as by Ni²⁺-chelate affinity chromatography.

Stable expression in CHO cells was performed using the following procedure. The proteins were expressed as an IgG construct (immunoadhesin), in which the coding sequences for the soluble forms (e.g. extracellular domains) of the respective proteins were fused to an IgG 1 constant region sequence containing the hinge, CH2 and CH2 domains and/or is a poly-His tagged form.

Following PCR amplification, the respective DNAs were subcloned in a CHO expression vector using standard techniques as described in Ausubel et al., *Current Protocols of Molecular Biology,* Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas *et al., Nucl. Acids Res.* 24: 9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA were introduced into approximately 10 million CHO cells using commercially available transfection reagents Superfect® (Quiagen), Dosper® or Fugene® (Boehringer Mannheim). The cells were grown and described in Lucas *et al.,* supra. Approximately 3 x 10⁻⁷ cells are frozen in an ampule for further growth and production as described below.

The ampules containing the plasmid DNA were thawed by placement into water bath and mixed by vortexing. The contents were pipetted into a centrifuge tube containing 10 mLs of media and centrifuged at 1000 rpm for 5 minutes. The supernatant was aspirated and the cells were resuspended in 10 mL of selective media (0.2 *µ*m filtered PS20 with 5% 0.2µm diafiltered fetal boyine serum). The cells were then aliquoted into a 100 mL spinner containing 90 mL of selective media. After 1-2 days, the cells were transferred into a 250 mL spinner filled with 150 mL selective growth medium and incubated at 37°C. After another 2-3 days, a 250 mL, 500 mL and 2000 mL spinners were seeded with 3 x 10⁵ cells/mL. The cell media was exchanged with fresh media by centrifugation and resuspension in production medium. Although any suitable CHO media may be employed, a production medium described in US Patent No. 5,122,469, issued June 16, 1992 was actually used. 3L production spinner is seeded at 1.2 x 10⁶ cells/mL. On day 0, the cell number pH were determined. On day 1, the spinner was sampled and sparging with filtered air was commenced. On day 2, the spinner was sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion). Throughout the production, pH was adjusted as necessary to keep at around 7.2. After 10 days, or until viability dropped below 70%, the cell culture was harvested by centrifugtion and filtering through a 0.22 *µ*m filter. The filtrate was either stored at 4°C or immediately loaded onto columns for purification.

For the poly-His tagged constructs, the proteins were purified using a Ni-NTA column (Qiagen). Before purification, imidazole was added to the conditioned media to a concentration of 5 mM. The conditioned media was pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column was washed with additional equilibration buffer and the protein eluted with equilibration buffer containing 0.25 M imidazole. The highly purified protein was subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc containing) constructs of were purified from the conditioned media as follows. The conditioned medium was pumped onto a 5 ml Protein A column (Pharmacia) which had been equilibrated in 20 mM Na phosphate buffer, pH 6.8. After loading, the column was washed extensively with equilibration buffer before elution with 100 mM citric acid, pH 3.5. The eluted protein was immediately neutralized by collecting 1 ml fractions into tubes containing 275 µL of 1 M Tris buffer, pH 9. The highly purified protein was subsequently desalted into storage buffer as described above for the poly-His tagged proteins. The homogeneity was assessed by SDS polyacrylamide gels and by N-terminal amino acid sequencing by Edman degradation.

The following PRO polypeptide: was successfully transiently expressed in COS cells: PRO181.

The following PRO polypeptide was successfully stably expressed in CHO cells: PRO181.

### EXAMPLE 7: Expression of PRO Polypeptides in Yeast

The following method describes recombinant expression of a desired PRO polypeptide in yeast.

First, yeast expression vectors are constructed for intracellular production or secretion of PRO polypeptides from the ADH2/GAPDH promoter. DNA encoding a desired PRO polypeptide, a selected signal peptide and the promoter is inserted into suitable restriction enzyme sites in the selected plasmid to direct intracellular expression of the PRO polypeptide. For secretion, DNA encoding the PRO polypeptide can be cloned into the selected plasmid, together with DNA encoding the ADH2/GAPDH promoter, the yeast alpha-factor secretory signal/leader sequence, and linker sequences (if needed) for expression of the PRO polypeptide.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE. followed by staining of the gels with Coomassie Blue stain.

Recombinant PRO polypeptide can subsequently be isolated and purified by removing the yeast cells from the fermentation medium by centrifugation and then concentrating the medium using selected cartridge filters. The concentrate containing the PRO polypeptide may further be purified using selected column chromatography resins.

### EXAMPLE 8: Expression of PRO Polypeptides in Baculovirus-Infected Insect Cells

The following method describes recombinant expression of PRO polypeptides in Baculovirus-infected insect cells.

The desired PRO polypeptide is fused upstream of an epitope tag contained with a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the PRO polypeptide or the desired portion of the PRO polypeptide (such as the sequence encoding the extracellular domain of a transmembrane protein) is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{™} virus DNA (Pharmingen) into *Spodoptera frugiperda* ("Sf9") cells (ATCC CRL 1711) using lipofectin (commercially available from GIBCO-BRL). After 4-5 days of incubation at 28°C, the released viruses are harvested and used for further amplifications. Viral infection and protein expression is performed as described by O'Reilley et al., Baculovirus expression vectors: A laboratory Manual, Oxford: Oxford University Press (1994).

Expressed poly-his tagged PRO polypeptide can then be purified, for example, by Ni²⁺-chelate affinity chromatography as follows. Extracts are prepared from recombinant virus-infected Sf9 cells as described by Rupert *et al., Nature,* 362:175-179 (1993). Briefly, Sf9 cells are washed, resuspended in sonication buffer (25 mL Hepes, pH 7.9; 12.5 mM MgCl₂; 0.1 mM EDTA; 10% Glycerol; 0.1 % NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% Glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% Glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or western blot with Ni²⁺-NTA-conjugated to alkaline phosphatase (Qiagen). Fractions containing the eluted His₁₀-tagged PRO polypeptide are pooled and dialyzed against loading buffer.

Alternatively, purification of the IgG tagged (or Fc tagged) PRO polypeptide can be performed using known chromatography techniques, including for instance, Protein A or protein G column chromatography.

In EP Application No. 99912321.9, polypeptides were successfully expressed in baculovirus infected Sf9 insect cells. While the expression was actually performed in a 0.5-2 L scale, it can be readily scaled up for larger (e.g. 8 L) preparations. The proteins were expressed as an IgG construct (immunoadhesin), in which the protein extracellular region was fused to an IgG1 constant region sequence containing the hinge, CH2 and CH3 domains and/or in poly-His tagged forms.

For expression in baculovirus infected Sf9 cells, following PCR amplification, the respective coding sequences were subcloned into a baculovirus expression vector (pb.PH.IgG for IgG fusions and pb.PH.His.c for poly-His tagged proteins), and the vector and Baculogold® baculovirus DNA (Pharmingen) were co-transfected into 105 *Spodopterafrugiperda* ("Sf9") cells (ATCC CRL 1711), using Lipofectin (Gibco BRL). pb.PH.IgG and pb.PH.His are modifications of the commercially available baculovirus expression vector pVL1393 (Pharmingen), with modified polylinker regions to include the His or Fc tag sequences. The cells were grown in Hink's TNM-FH medium supplemented with 10% FBS (Hyclone). Cells were incubated for 5 days at 28°C. The supernatant was harvested and subsequently used for the first viral amplification by infecting Sf9 cells in Hink's TNM-FH medium supplemented with 10% FBS at an approximate multiplicity of infection (MOI) of 10. Cells were incubated for 3 days at 28°C. The supernatant was harvested and the expression of the constructs in the baculovirus expression vector was determined by batch binding of 1 ml of supernatant to 25 mL of Ni-NTA beads (QIAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The first viral amplification supernatant was used to infect a spinner culture (500 ml) of Sf9 cells grown in ESF-921 medium (Expression Systems LLC) at an approximate MOI of 0.1. Cells were incubated for 3 days at 28°C. The supernatant was harvested and filtered. Batch binding and SDS-PAGE analysis was repeated, as necessary, until expression of the spinner culture was confirmed.

The conditioned medium from the transfected cells (0.5 to 3 L) was harvested by centrifugation to remove the cells and filtered through 0.22 micron filters. For the poly-His tagged constructs, the protein construct were purified using a Ni-NTA column (Qiagen). Before purification, imidazole was added to the conditioned media to a concentration of 5 mM. The conditioned media were pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column was washed with additional equilibration buffer and the protein eluted with equilibration buffer containing 0.25 M imidazole. The highly purified protein was subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc containing) constructs of proteins were purified from the conditioned media as follows. The conditioned media were pumped onto a 5 ml Protein A column (Pharmacia) which had been equilibrated in 20 mM Na phosphate buffer, pH 6.8. After loading, the column was washed extensively with equilibration buffer before elution with 100 mM citric acid, pH 3.5. The eluted protein was immediately neutralized by collecting 1 ml fractions into tubes containing 275 mL of 1 M Tris buffer, pH 9. The highly purified protein was subsequently desalted into storage buffer as described above for the poly-His tagged proteins. The homogeneity of the proteins was verified by SDS polyacrylamide gel (PEG) electrophoresis and N-terminal amino acid sequencing by Edman degradation.

PRO181 was successfully expressed in baculovirus infected Hi5 insect ceits. While the expression was actually performed in a 0.5-2 L scale, it can be readily scaled up for larger (e.g. 8 L) preparations.

For expression in baculovirus-infected Hi5 insect cells, the PRO polypeptide-encoding DNA may be amplified with suitable systems, such as Pfu (Stratagene), or fused upstream (5'-of) of an epitope tag contained with a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the PRO polypeptide or the desired portion of the PRO polypeptide (such as the sequence encoding the extracellular domain of a transmembrane protein) is amplified by PCR with primers complementary to the 5' and 3' regions. The 5' primer may incorporate flanking (selected) restriction enzyme sites. The product is then digested with those selected restriction enzymes and subcloned into the expression vector. For example, derivatives of pVL1393 can include the Fc region of human IgG (pb.PH.IgG) or an 8 histidine (pb.PH.His) tag downstream (3'-of) the NAME sequence. Preferably, the vector construct is sequenced for confirmation.

Hi5 cells are grown to a confluency of 50% under the conditions of, 27°C, no CO2, NO pen/strep. For each 150 mm plate, 30 ug of pIE based vector containing PRO polypeptide is mixed with 1 ml Ex-Cell medium (Media: Ex-Cell 401 + 1/100 L-Glu JRH Biosciences #14401-78P (note: this media is light sensitive)), and in a separate tube, 100 ul of CellFectin (CellFECTIN (GibcoBRL #10362-010) (vortexed to mix)) is mixed with 1 ml of Ex-Cell medium. The two solutions are combined and allowed to incubate at room temperature for 15 minutes. 8 ml of Ex-Cell media is added to the 2m1 of DNA/CellFECTIN mix and this is layered on Hi5 cells that have been washed once with Ex-Cell media. The plate is then incubated in darkness for 1 hour at room temperature. The DNA/CellFECTIN mix is then aspirated, and the cells are washed once with Ex-Cell to remove excess CellFECTIN . 30 ml of fresh Ex-Cell media is added and the cells are incubated for 3 days at 28°C. The supernatant is harvested and the expression of the PRO polypeptide in the baculovirus expression vector can be determined by batch binding of 1 ml of supernatent to 25 mL of Ni-NTA beads (QIAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The conditioned media from the transfected cells (0.5 to 3 L) is harvested by centrifugation to remove the cells and filtered through 0.22 micron filters. For the poly-His tagged constructs, the protein comprising the PRO polypeptide is purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently deslated into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc containing) constructs of proteins are purified from the conditioned media as follows. The conditioned media is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 mL of 1 M Tris buffer, pH 9. The highly purified protein is subsequently desalted into storage buffer as described above for the poly-His tagged proteins. The homogeneity of PRO polypeptide can be assessed by SDS polyacrylatnide gels and by N-terminal amino acid sequencing by Edman degradation and other analytical procedures as desired or necessary.

### EXAMPLE 9: Preparation of Antibodies that Bind to PRO Polypeptides

This example illustrates preparation of monoclonal antibodies which can specifically bind to a PRO polypeptide.

Techniques for producing the monoclonal antibodies are known in the art and are described, for instance, in Goding, supra. Immunogens that may be employed include purified PRO polypeptide, fusion proteins containing the PRO polypeptide, and cells expressing recombinant PRO polypeptide on the cell surface. Selection of the immunogen can be made by the skilled artisan without undue experimentation.

Mice, such as Balb/c, are immunized with the PRO polypeptide immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO polypeptide antibodies.

After a suitable antibody titer has been detected, the animals "positive" for antibodies can be injected with a final intravenous injection of PRO polypeptide. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against the PRO polypeptide. Determination of "positive" hybridoma cells secreting the desired monoclonal antibodies against the PRO polypeptide is within the skill in the art.

The positive hybridoma cells can be injected intraperitoneally into syngeneic Balb/c mice to produce ascites containing the anti-PRO polypeptide monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 10: Chimeric PRO Polypeptides

PRO polypeptides may be expressed as chimeric proteins with one or more additional polypeptide domains added to facilitate protein purification. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS^{™} extension/affinity purification system (Immunex Corp., Seattle Wash.). The inclusion of a cleavable linker sequence such as Factor XA or enterokinase (Invitrogen, San Diego Calif.) between the purification domain and the PRO polypeptide sequence may be useful to facilitate expression of DNA encoding the PRO polypeptide.

### EXAMPLE 11: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE^{™} (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide *(e.g.,* high ionic strength buffers in the presence of detergent). Then, the column is eluted under conditions that disrupt antibody/PRO polypeptide binding (*e.g.*, a low pH buffer such as approximately pH 2-3, or a high concentration of a chaotrope such as urea or thiocyanate ion), and PRO polypeptide is collected.

### EXAMPLE 12: Drug Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (I) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

### EXAMPLE 13: Rational Drug Design

The goal of rational drug design is to produce structural analogs of biologically active polypeptide of interest (*i.e.*, a PRO polypeptide) or of small molecules with which they interact, *e.g*., agonists, antagonists, or inhibitors. Any of these examples can be used to fashion drugs which are more active or stable forms of the PRO polypeptide or which enhance or interfere with the function of the PRO polypeptide *in vivo (c.f.,* Hodgson, Bio/Technology, 9: 19-21 (1991)).

In one approach, the three-dimensional structure of the PRO polypeptide, or of an PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by *Athauda et al.,* J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

### EXAMPLE 14: In Vitro Antiproliferative Assay

The antiproliferative activity of various PRO polypeptides was determined in the investigational, disease-oriented *in vitro* anti-cancer drug discovery assay of the National Cancer Institute (NCI), using a sulforhodamine B (SRB) dye binding assay essentially as described by Skehan et al., *J. Natl. Cancer Inst.* 82:1107-1112 (1990). The 60 tumor cell lines employed in this study ("the NCl panel"), as well as conditions for their maintenance and culture *in vitro* have been described by Monks et al., *J*. *Natl. Cancer Inst.* 83:757-766 (1991). The purpose of this screen is to initially evaluate the cytotoxic and/or cytostatic activity of the test compounds against different types of tumors (Monks et al., *supra;* Boyd, *Cancer: Princ. Pract. Oncol. Update* 3(10):1-12 [1989]).

Cells from approximately 60 human tumor cell lines were harvested with trypsin/EDTA (Gibco), washed once, resuspended in IMEM and their viability was determined. The cell suspensions were added by pipet (100 *µ*L volume) into separate 96-well microtiter plates. The cell density for the 6-day incubation was less than for the 2-day incubation to prevent overgrowth. Inoculates were allowed a preincubation period of 24 hours at 37°C for stabilization. Dilutions at twice the intended test concentration were added at time zero in 100 *µ*L aliquots to the microtiter plate wells (1:2 dilution). Test compounds were evaluated at five half-log dilutions (1000 to 100,000-fold). Incubations took place for two days and six days in a 5% CO₂ atmosphere and 100% humidity.

After incubation, the medium was removed and the cells were fixed in 0.1 ml of 10% trichloroacetic acid at 40°C. The plates were rinsed five times with deionized water, dried, stained for 30 minutes with 0.1 ml of 0.4% sulforhodamine B dye (Sigma) dissolved in 1 % acetic acid, rinsed four times with I % acetic acid to remove unbound dye, dried, and the stain was extracted for five minutes with 0.1 ml of 10 mM Tris base [tris(hydroxymethyl)aminomethane], pH 10.5. The absorbance (OD) of sulforhodamine B at 492 nm was measured using a computer-interfaced, 96-well microtiter plate reader.

A test sample is considered positive if it shows at least 50% growth inhibitory effect at one or more concentrations. The following PRO polypeptide gave positive results in at least one tumor cell line: PRO181.

### EXAMPLE 15: In situ Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis and aid in chromosome mapping.

*In situ* hybridization was performed following an optimized version of the protocol by Lu and Gillett, Cell Vision 1:169-176 (1994), using PCR-generated ³³P-labeled riboprobes. Briefly, formalin-fixed, paraffin-embedded human tissues were sectioned, deparaffinized, deproteinated in proteinase K (20 g/ml) for 15 minutes at 37°C, and further processed for *in situ* hybridization as described by Lu and Gillett, *supra.* A [³³-P] UTP-labeled antisense riboprobe was generated from a PCR product and hybridized at 55°C overnight. The slides were dipped in Kodak NTB2 nuclear track emulsion and exposed for 4 weeks.

### ³³P-Riboprobe synthesis

6.0 µl (125 mCi) of ³³P-UTP (Amersham BF 1002, SA < 2000 Ci/mmol) were speed vac dried. To each tube containing dried ³³P-UTP, the following ingredients were added:
2.0 µl 5x transcription buffer
1.0 µl DTT (100 mM)
2.0 µl NTP mix (2.5 mM : 10 *µ;* each of 10 mM GTP, CTP & ATP + 10 µl H₂O)
1.0 µl UTP (50 µM
1.0 µl Rnasin
1.0 µl DNA template (1µg)
1.0 µl H₂O
1.0 µl RNA polymerase (for PCR products T3 = AS, T7 = S, usually)

The tubes were incubated at 37°C for one hour. 1.0 µl RQ1 DNase were added, followed by incubation at 37°C for 15 minutes. 90 µl TE (10 mM Tris pH 7.6/1mM EDTA pH 8.0) were added, and the mixture was pipetted onto DE81 paper. The remaining solution was loaded in a Microcon-50 ultrafiltration unit, and spun using program 10 (6 minutes). The filtration unit was inverted over a second tube and spun using program 2 (3 minutes). After the final recovery spin, 100 µl TE were added. 1 µl of the final product was pipetted on DE81 paper and counted in 6 ml of Biofluor II.

The probe was run on a TBE/urea gel. 1-3 µl of the probe or 5 µl of RNA Mrk III were added to 3 µl of loading buffer. After heating on a 95°C heat block for three minutes, the gel was immediately placed on ice. The wells of gel were flushed, the sample loaded, and run at 180-250 volts for 45 minutes. The gel was wrapped in saran wrap and exposed to XAR film with an intensifying screen in -70°C freezer one hour to overnight. ^{**33**}**P-Hybridization**

### A. Pretreatment of frozen sections

The slides were removed from the freezer, placed on aluminium trays and thawed at room temperature for 5 minutes. The trays were placed in 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ H₂O). After deproteination in 0.5 µg/ml proteinase K for 10 minutes at 37°C (12.5 µl of 10 mg/ml stock in 250 ml prewarmed RNase-free RNAse buffer), the sections were washed in 0.5 x SSC for 10 minutes at room temperature. The sections were dehydrated in 70%, 95%, 100% ethanol, 2 minutes each.

### B. Pretreatment of paraffin-embedded sections

The slides were deparaffinized, placed in SQ H₂O, and rinsed twice in 2 x SSC at room temperature, for 5 minutes each time. The sections were deproteinated in 20 µg/ml proteinase K (500 µl of 10 mg/ml in 250 ml RNase-free RNase buffer; 37°C, 15 minutes) - human embryo, or 8 x proteinase K (100µl in 250 ml Rnase buffer, 37°C, 30 minutes) - formalin tissues. Subsequent rinsing in 0.5 x SSC and dehydration were performed as described above.

### C. Prehybridization

The slides were laid out in a plastic box lined with Box buffer (4 x SSC, 50% formamide) - saturated filter paper. The tissue was covered with 50 *µ*l of hybridization buffer (3.75g Dextran Sulfate + 6 ml SQ H₂O), vortexed and heated in the microwave for 2 minutes with the cap loosened. After cooling on ice, 18.75 ml formamide, 3.75 ml 20 x SSC and 9 ml SQ H₂O were added, the tissue was vortexed well, and incubated at 42°C for 1-4 hours.

### D. Hybridization

1.0 x 10⁶ cpm probe and 1.0 *µ*l tRNA (50 mg/ml stock) per slide were heated at 95°C for 3 minutes. The slides were cooled on ice, and 48 *µ*l hybridization buffer were added per slide. After vortexing, 50 *µ*l ³³P mix were added to 50 *µ*l prehybridization on slide. The slides were incubated overnight at 55°C.

### E. Washes

Washing was done 2 x 10 minutes with 2xSSC, EDTA at room temperature (400 ml 20 x SSC + 16 ml 0.25M EDTA, V_{f}=4L), followed by RNaseA treatment at 37°C for 30 minutes (500 µl of 10 mg/ml in 250 ml Rnase buffer = 20 µg/ml), The slides were washed 2 x 10 minutes with 2 x SSC, EDTA at room temperature. The stringency wash conditions were as follows: 2 hours at 55°C, 0.1 x SSC, EDTA (20 ml 20 x SSC + 16 ml EDTA, V_{f}=4L).

### F. Oligonucleotides

*In situ* analysis was performed on a variety of DNA sequences disclosed in EP 99912321.9. The oligonucleotides employed for these analyses were derived from the nucleotide sequences disclosed in EP 99912321.9. and generally range from about 40 to 55 nucleotides in length.

### Deposit of Material

The following materials have been deposited with the American Type Culture Collection, 12301 Parklawn Drive, Rockville, MD, USA (ATCC):

| Material | ATCC Dep. No. | Deposit Date |
|---|---|---|
| DNA23330-1390 | ATCC 209775 | April 14, 1998 |

These deposit were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposits will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC § 122 and the Commissioner's rules pursuant thereto (including 37 CFR § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention.

The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications in addition to those shown and described herein will become apparent to those skilled in the art from the foregoing description and fall within the scope of the appended claims.

## Claims

1. Isolated polypeptide for use in a method of medical treatment, wherein the polypeptide:
(a) has at least 80% sequence identity to the amino acid sequence shown in Figure 2 (SEQ ID NO:2), wherein the sequence identity is determined over the full length of the amino acid sequence of Fig. 2 (SEQ ID NO:2),
(b) comprises residues 1 or 21 to X of the amino acid sequence shown in Figure 2 (SEQ ID NO:2), wherein X is any residue from 52 to 61, or X is 144,
(c) comprises an extracellular domain of the amino acid sequence of Fig. 2, having the sequence of amino acids 32±5 to 56±5,
(d) has at least 80% sequence identity to the amino acid sequence encoded by the full-length coding sequence of the nucleic acid insert of the vector deposited as accession number ATCC 209775, wherein the sequence identity is determined over the full length of the encoded amino acid sequence, or
(e) consists of the amino acid sequence encoded by the full-length coding sequence of the nucleic acid insert of the vector deposited as accession number ATCC 209775.

2. The polypeptide of Claim 1 for use in a method of medical treatment, herein the level of identity is at least 90%.

3. The polypeptide of Claim 2 for use in a method of medical treatment, wherein the level of identity is at least 95%.

4. The polypeptide of any preceding claim for use in a method of medical treatment, wherein the treatment is of tumour.

5. The use of a polypeptide according to any one of Claims 1 to 3 in the manufacture of a medicament for the treatment of tumour.

## Patentansprüche

1. Isoliertes Polypeptid zur Verwendung in einem medizinischen Behandlungsverfahren, worin das Polypeptid:
(a) zumindest 80 % Sequenzidentität mit der in Fig. 2 gezeigten Aminosäuresequenz (Seq.-ID Nr. 2) aufweist, worin die Sequenzidentität über die volle Länge der Aminosäuresequenz aus Fig. 2 (Seq.-ID Nr. 2) bestimmt wird,
(b) die Reste 1 oder 21 bis X der in Fig. 2 gezeigten Aminosäuresequenz (Seq.-ID Nr. 2) umfasst, worin X ein beliebiger Rest von 52 bis 61 ist oder X 144 ist,
(c) eine extrazelluläre Domäne der Aminosäuresequenz aus Fig. 2 mit der Sequenz der Aminosäuren 32±5 bis 56±5 umfasst,
(d) zumindest 80 % Sequenzidentität mit der Aminosäuresequenz aufweist, für die die Volllängenkodiersequenz des Nucleinsäureinserts des unter der Zugriffsnummer ATCC 209775 hinterlegten Vektors kodiert, worin die Sequenzidentität über die volle Länge der kodierten Aminosäuresequenz bestimmt wird, oder
(e) aus der Aminosäuresequenz besteht, für die die Volllängenkodiersequenz des Nucleinsäureinserts des unter der Zugriffsnummer ATCC 209775 hinterlegten Vektors kodiert.

2. Polypeptid nach Anspruch 1 zur Verwendung in einem medizinischen Behandlungsverfahren, worin sich der Identitätsgrad auf zumindest 90 % beläuft.

3. Polypeptid nach Anspruch 2 zur Verwendung in einem medizinischen Behandlungsverfahren, worin sich der Identitätsgrad auf zumindest 95 % beläuft.

4. Polypeptid nach einem der vorangehenden Ansprüche zur Verwendung in einem medizinischen Behandlungsverfahren, worin die Behandlung jene von Tumoren ist.

5. Verwendung eines Polypeptid nach einem der Ansprüche 1 bis 3 bei der Herstellung eines Medikaments zur Behandlung von Tumoren.

## Revendications

1. Polypeptide isolé destiné à être utilisé dans une méthode de traitement médical, ledit polypeptide :
(a) ayant une identité de séquence d'au moins 80 % avec la séquence d'aminoacides représentée sur la figure 2 (SEQ ID N° 2), l'identité de séquence étant déterminée sur la longueur totale de la séquence d'aminoacides de la figure 2 (SEQ ID N° 2),
(b) comprenant les résidus 1 ou 21 à X de la séquence d'aminoacides représentée sur la figure 2 (SEQ ID N° 2), où X représente n'importe quel résidu de 52 à 61, ou bien X représente le résidu 144,
(c) comprenant un domaine excracellulaire de la séquence d'aminoacides de la figure 2 ayant la séquence d'aminoacides 32 ± 5 à 56 ± 5,
(d) ayant une identité de séquence d'au moins 80 % avec la séquence d'aminoacides codée par la séquence codante totale du segment d'insertion d'acide nucléique du vecteur déposé sous le numéro de dépôt ATCC 209775, l'identité de séquence étant déterminée sur la longueur totale de la séquence d'aminoacides codée ou
(e) consistant en la séquence d'aminoacides codée par la séquence codante totale du segment d'insertion d'acide nucléique du vecteur déposé sous le numéro de dépôt ATCC 209775.

2. Polypeptide suivant la revendication 1, destiné à être utilisé dans une méthode de traitement médical, où le degré d'identité est d'au moins 90 %.

3. Polypeptide suivant la revendication 2, destiné à être utilisé dans une méthode de traitement médical, où le degré d'identité est d'au mains 95 %.

4. Polypeptide suivant l'une quelconque des revendications précédentes, destiné à être utilisé dans une méthode de traitement médical, dans lequel le traitement est le traitement d'une tumeur.

5. Utilisation d'un polypeptide suivant l'une quelconque des revendications 1 à 3, dans la production d'un médicament destiné au traitement d'une tumeur.


### Sequence Listing

<110> Genentech, Inc.
<120> Novel Polypeptides and Nucleic Acids Encoding the Same
<130> CMD/FP5979448
<140> EP 02012896.3
   <141> 2002-06-11
<150> EP 99912321.9
   <151> 1999-03-08
<150> PCT/US99/05028
   <151> 1999-03-08
<150> US 60/081,955
   <151> 1998-04-15
<160> 8
<210> 1
   <211> 1333
   <212> DNA
   <213> Homo sapiens
<400> 1
   gcccacgcgt ccgatggcgt tcacgttcgc ggccttctgc tacatgctgg 50
   cgctgctgct cactgccgcg ctcatcttct tcgccatttg gcacattata 100
   gcatttgatg agctgaagac tgattacaag aatcctatag accagtgtaa 150
   taccctgaat ccccttgtac tcccagagta cctcatccac gctttcttct 200
   gtgtcatgtt tctttgtgca gcagagtggc ttacactggg tctcaatatg 250
   cccctcttgg catatcatat ttggaggtat atgagtagac cagtgatgag 300
   tggcccagga ctctatgacc ctacaaccat catgaatgca gatattctag 350
   catattgtca gaaggaagga tggtgcaaat tagcttttta tcttctagca 400
   tttttttact acctatatgg catgatctat gttttggtga gctcttagaa 450
   caacacacag aagaattggt ccagttaagt gcatgcaaaa agccaccaaa 500
   tgaagggatt ctatccagca agatcctgtc caagagtagc ctgtggaatc 550
   tgatcagtta ctttaaaaaa tgactcctta ttttttaaat gtttccacat 600
   ttttgcttgt ggaaagactg ttttcatatg ttatactcag ataaagattt 650
   taaatggtat tacgtataaa ttaatataaa atgattacct ctggtgttga 700
   caggtttgaa cttgcacttc ttaaggaaca gccataatcc tctgaatgat 750
   gcattaatta ctgactgtcc tagtacattg gaagcttttg tttataggaa 800
   cttgtagggc tcattttggt ttcattgaaa cagtatctaa ttataaatta 850
   gctgtagata tcaggtgctt ctgatgaagt gaaaatgtat atctgactag 900
   tgggaaactt catgggtttc ctcatctgtc atgtcgatga ttatatatgg 950
   atacatttac aaaaataaaa agcgggaatt ttcccttcgc ttgaatatta 1000
   tccctgtata ttgcatgaat gagagatttc ccatatttcc atcagagtaa 1050
   taaatatact tgctttaatt cttaagcata agtaaacatg atataaaaat 1100
   atatgctgaa ttacttgtga agaatgcatt taaagctatt ttaaatgtgt 1150
   ttttatttgt aagacattac ttattaagaa attggttatt atgcttactg 1200
   ttctaatctg gtggtaaagg tattcttaag aatttgcagg tactacagat 1250
   tttcaaaact gaatgagaga aaattgtata accatcctgc tgttccttta 1300
   gtgcaataca ataaaactct gaaattaaga ctc 1333
<210> 2
   <211> 144
   <212> PRT
   <213> Homo sapiens
<400> 2
   Met Ala Phe Thr Phe Ala Ala Phe Cys Tyr Met Leu Ala Leu Leu
   1 5 10 15
   Leu Thr Ala Ala Leu Ile Phe Phe Ala Ile Trp His Ile Ile Ala
   20 25 30
   Phe Asp Glu Leu Lys Thr Asp Tyr Lys Asn Pro Ile Asp Gln Cys
   35 40 45
   Asn Thr Leu Asn Pro Leu Val Leu Pro Glu Tyr Leu Ile His Ala
   50 55 60
   Phe Phe Cys Val Met Phe Leu Cys Ala Ala Glu Trp Leu Thr Leu
   65 70 75
   Gly Leu Asn Met Pro Leu Leu Ala Tyr His Ile Trp Arg Tyr Met
   80 85 90
   Ser Arg Pro Val Met Ser Gly Pro Gly Leu Tyr Asp Pro Thr Thr
   95 100 105
   Ile Met Asn Ala Asp Ile Leu Ala Tyr Cys Gln Lys Glu Gly Trp
   110 115 120
   Cys Lys Leu Ala Phe Tyr Leu Leu Ala Phe Phe Tyr Tyr Leu Tyr
   125 130 135
   Gly Met Ile Tyr Val Leu Val Ser Ser
   140
<210> 3
   <211> 477
   <212> DNA
   <213> Homo sapiens
<400> 3
   attatagcat ttgatgagct gaagactgat tacaagatcc tatagaccag 50
   tgtaataccc tgaatcccct tgtactccca gagtacctca tccacgcttt 100
   cttctgtgtc atgtttcttt gtgcagcaga gtggcttaca ctgggtctca 150
   atatgcccct cttggcatat catatttgga ggtatatgag tagaccagtg 200
   atgagtggcc caggactcta tgaccctaca accatcatga atgcagatat 250
   tctagcatat tgtcagaagg aaggatggtg caaattagct ttttatcttc 300
   tagcattttt ttactaccta tatggcatga tctatgtttt ggtgagctct 350
   tagaacaaca cacagaagaa ttggtccagt taagtgcatg caaaaagcca 400
   ccaaatgaag ggattctatc cagcaagatc ctgtccaaga gtagcctgtg 450
   gaatctgatc agttacttta aaaaatg 477
<210> 4
   <211> 43
   <212> DNA
   <213> Artificial
<220>
   <223> Artificial Sequence
<400> 4
   tgtaaaacga cggccagtta aatagacctg caattattaa tct 43
<210> 5
   <211> 41
   <212> DNA
   <213> Artificial
<220>
   <223> Artificial Sequence
<400> 5
   caggaaacag ctatgaccac ctgcacacct gcaaatccat t 41
<210> 6
   <211> 20
   <212> DNA
   <213> Artificial
<220>
   <223> Artificial Sequence
<400> 6
   gtgcagcaga gtggcttaca 20
<210> 7
   <211> 20
   <212> DNA
   <213> Artificial
<220>
   <223> Artificial Sequence
<400> 7
   actggaccaa ttcttctgtg 20
<210> 8
   <211> 45
   <212> DNA
   <213> Artificial
<220>
   <223> Artificial Sequence
<400> 8
   gatattctag catattgtca gaaggaagga tggtgcaaat tagct 45

### Sequence Listing

<110> Genentech, Inc.
<120> Novel Polypeptides and Nucleic Acids Encoding the Same
<130> CMD/FP5979448
<140> EP 02012896.3
   <141> 2002-06-11
<150> EP 99912321.9
   <151> 1999-03-08
<150> PCT/US99/05028
   <151> 1999-03-08
<150> US 60/081,955
   <151> 1998-04-15
<160> 8
<210> 1
   <211> 1333
   <212> DNA
   <213> Homo sapiens
<400> 1
   gcccacgcgt ccgatggcgt tcacgttcgc ggccttctgc tacatgctgg 50
   cgctgctgct cactgccgcg ctcatcttct tcgccatttg gcacattata 100
   gcatttgatg agctgaagac tgattacaag aatcctatag accagtgtaa 150
   taccctgaat ccccttgtac tcccagagta cctcatccac gctttcttct 200
   gtgtcatgtt tctttgtgca gcagagtggc ttacactggg tctcaatatg 250
   cccctcttgg catatcatat ttggaggtat atgagtagac cagtgatgag 300
   tggcccagga ctctatgacc ctacaaccat catgaatgca gatattctag 350
   catattgtca gaaggaagga tggtgcaaat tagcttttta tcttctagca 400
   tttttttact acctatatgg catgatctat gttttggtga gctcttagaa 450
   caacacacag aagaattggt ccagttaagt gcatgcaaaa agccaccaaa 500
   tgaagggatt ctatccagca agatcctgtc caagagtagc ctgtggaatc 550
   tgatcagtta ctttaaaaaa tgactcctta ttttttaaat gtttccacat 600
   ttttgcttgt ggaaagactg ttttcatatg ttatactcag ataaagattt 650
   taaatggtat tacgtataaa ttaatataaa atgattacct ctggtgttga 700
   caggtttgaa cttgcacttc ttaaggaaca gccataatcc tctgaatgat 750
   gcattaatta ctgactgtcc tagtacattg gaagcttttg tttataggaa 800
   cttgtagggc tcattttggt ttcattgaaa cagtatctaa ttataaatta 850
   gctgtagata tcaggtgctt ctgatgaagt gaaaatgtat atctgactag 900
   tgggaaactt catgggtttc ctcatctgtc atgtcgatga ttatatatgg 950
   atacatttac aaaaataaaa agcgggaatt ttcccttcgc ttgaatatta 1000
   tccctgtata ttgcatgaat gagagatttc ccatatttcc atcagagtaa 1050
   taaatatact tgctttaatt cttaagcata agtaaacatg atataaaaat 1100
   atatgctgaa ttacttgtga agaatgcatt taaagctatt ttaaatgtgt 1150
   ttttatttgt aagacattac ttattaagaa attggttatt atgcttactg 1200
   ttctaatctg gtggtaaagg tattcttaag aatttgcagg tactacagat 1250
   tttcaaaact gaatgagaga aaattgtata accatcctgc tgttccttta 1300
   gtgcaataca ataaaactct gaaattaaga ctc                   1333
<210> 2
   <211> 144
   <212> PRT
   <213> Homo sapiens
<400> 2
   Met Ala Phe Thr Phe Ala Ala Phe Cys Tyr Met Leu Ala Leu Leu
   1               5                   10                  15
   Leu Thr Ala Ala Leu Ile Phe Phe Ala Ile Trp His Ile Ile Ala
                   20                  25                  30
   Phe Asp Glu Leu Lys Thr Asp Tyr Lys Asn Pro Ile Asp Gln Cys
                   35                  40                  45
   Asn Thr Leu Asn Pro Leu Val Leu Pro Glu Tyr Leu Ile His Ala
                   50                  55                  60
   Phe Phe Cys Val Met Phe Leu Cys Ala Ala Glu Trp Leu Thr Leu
                   65                  70                  75
   Gly Leu Asn Met Pro Leu Leu Ala Tyr His Ile Trp Arg Tyr Met
                   80                  85                  90
   Ser Arg Pro Val Met Ser Gly Pro Gly Leu Tyr Asp Pro Thr Thr
                   95                  100                 105
   Ile Met Asn Ala Asp Ile Leu Ala Tyr Cys Gln Lys Glu Gly Trp
                   110                 115                 120
   Cys Lys Leu Ala Phe Tyr Leu Leu Ala Phe Phe Tyr Tyr Leu Tyr
                   125                 130                 135
   Gly Met Ile Tyr Val Leu Val Ser Ser
                   140
<210> 3
   <211> 477
   <212> DNA
   <213> Homo sapiens
<400> 3
   attatagcat ttgatgagct gaagactgat tacaagatcc tatagaccag 50
   tgtaataccc tgaatcccct tgtactccca gagtacctca tccacgcttt 100
   cttctgtgtc atgtttcttt gtgcagcaga gtggcttaca ctgggtctca 150
   atatgcccct cttggcatat catatttgga ggtatatgag tagaccagtg 200
   atgagtggcc caggactcta tgaccctaca accatcatga atgcagatat 250
   tctagcatat tgtcagaagg aaggatggtg caaattagct ttttatcttc 300
   tagcattttt ttactaccta tatggcatga tctatgtttt ggtgagctct 350
   tagaacaaca cacagaagaa ttggtccagt taagtgcatg caaaaagcca 400
   ccaaatgaag ggattctatc cagcaagatc ctgtccaaga gtagcctgtg 450
   gaatctgatc agttacttta aaaaatg 477
<210> 4
   <211> 43
   <212> DNA
   <213> Artificial
<220>
   <223> Artificial Sequence
<400> 4
   tgtaaaacga cggccagtta aatagacctg caattattaa tct 43
<210> 5
   <211> 41
   <212> DNA
   <213> Artificial
<220>
   <223> Artificial Sequence
<400> 5
   caggaaacag ctatgaccac ctgcacacct gcaaatccat t 41
<210> 6
   <211> 20
   <212> DNA
   <213> Artificial
<220>
   <223> Artificial Sequence
<400> 6
   gtgcagcaga gtggcttaca 20
<210> 7
   <211> 20
   <212> DNA
   <213> Artificial
<220>
   <223> Artificial Sequence
<400> 7
   actggaccaa ttcttctgtg 20
<210> 8
   <211> 45
   <212> DNA
   <213> Artificial
<220>
   <223> Artificial Sequence
<400> 8
   gatattctag catattgtca gaaggaagga tggtgcaaat tagct 45

### Sequence Listing

<110> Genentech, Inc.
<120> Novel Polypeptides and Nucleic Acids Encoding the Same
<130> CMD/FP5979448
<140> EP 02012896.3
   <141> 2002-06-11
<150> EP 99912321.9
   <151> 1999-03-08
<150> PCT/US99/05028
   <151> 1999-03-08
<150> US 60/081,955
   <151> 1998-04-15
<160> 328
<210> 1
<400> 1
   000
<210> 2
<400> 2
   000
<210> 3
<400> 3
   000
<210> 4
<400> 4
   000
<210> 5
<400> 5
   000
<210> 6
<400> 6
   000
<210> 7
<400> 7
   000
<210> 8
<400> 8
   000
<210> 9
<400> 9
   000
<210> 10
<400> 10
   000
<210> 11
<400> 11
   000
<210> 12
<400> 12
   000
<210> 13
<400> 13
   000
<210> 14
<400> 14
   000
<210> 15
<400> 15
   000
<210> 16
<400> 16
   000
<210> 17
<400> 17
   000
<210> 18
<400> 18
   000
<210> 19
<400> 19
   000
<210> 20
<400> 20
   000
<210> 21
<400> 21
   000
<210> 22
<400> 22
   000
<210> 23
<400> 23
   000
<210> 24
<400> 24
   000
<210> 25
<400> 25
   000
<210> 26
<400> 26
   000
<210> 27
<400> 27
   000
<210> 28
<400> 28
   000
<210> 29
<400> 29
   000
<210> 30
<400> 30
   000
<210> 31
<400> 31
   000
<210> 32
<400> 32
   000
<210> 33
<400> 33
   000
<210> 34
<400> 34
   000
<210> 35
<400> 35
   000
<210> 36
<400> 36
   000
<210> 37
<400> 37
   000
<210> 38
<400> 38
   000
<210> 39
<400> 39
   000
<210> 40
<400> 40
   000
<210> 41
<400> 41
   000
<210> 42
<400> 42
   000
<210> 43
<400> 43
   000
<210> 44
<400> 44
   000
<210> 45
<400> 45
   000
<210> 46
<400> 46
   000
<210> 47
<400> 47
   000
<210> 48
<400> 48
   000
<210> 49
<400> 49
   000
<210> 50
<400> 50
   000
<210> 51
<400> 51
   000
<210> 52
<400> 52
   000
<210> 53
<400> 53
   000
<210> 54
<400> 54
   000
<210> 55
<400> 55
   000
<210> 56
<400> 56
   000
<210> 57
<400> 57
   000
<210> 58
<400> 58
   000
<210> 59
<400> 59
   000
<210> 60
<400> 60
   000
<210> 61
<400> 61
   000
<210> 62
<400> 62
   000
<210> 63
<400> 63
   000
<210> 64
<400> 64
   000
<210> 65
<400> 65
   000
<210> 66
<400> 66
   000
<210> 67
<400> 67
   000
<210> 68
<400> 68
   000
<210> 69
<400> 69
   000
<210> 70
<400> 70
   000
<210> 71
<400> 71
   000
<210> 72
<400> 72
   000
<210> 73
<400> 73
   000
<210> 74
<400> 74
   000
<210> 75
<400> 75
   000
<210> 76
<400> 76
   000
<210> 77
<400> 77
   000
<210> 78
<400> 78
   000
<210> 79
<400> 79
   000
<210> 80
<400> 80
   000
<210> 81
<400> 81
   000
<210> 82
<400> 82
   000
<210> 83
<400> 83
   000
<210> 84
<400> 84
   000
<210> 85
<400> 85
   000
<210> 86
<400> 86
   000
<210> 87
<400> 87
   000
<210> 88
<400> 88
   000
<210> 89
<400> 89
   000
<210> 90
<400> 90
   000
<210> 91
<400> 91
   000
<210> 92
<400> 92
   000
<210> 93
<400> 93
   000
<210> 94
<400> 94
   000
<210> 95
<400> 95
   000
<210> 96
<400> 96
   000
<210> 97
<400> 97
   000
<210> 98
<400> 98
   000
<210> 99
<400> 99
   000
<210> 100
<400> 100
   000
<210> 101
<400> 101
   000
<210> 102
<400> 102
   000
<210> 103
<400> 103
   000
<210> 104
<400> 104
   000
<210> 105
<400> 105
   000
<210> 106
<400> 106
   000
<210> 107
<400> 107
   000
<210> 108
<400> 108
   000
<210> 109
<400> 109
   000
<210> 110
<400> 110
   000
<210> 111
<400> 111
   000
<210> 112
<400> 112
   000
<210> 113
<400> 113
   000
<210> 114
<400> 114
   000
<210> 115
<400> 115
   000
<210> 116
<400> 116
   000
<210> 117
<400> 117
   000
<210> 118
<400> 118
   000
<210> 119
<400> 119
   000
<210> 120
<400> 120
   000
<210> 121
<400> 121
   000
<210> 122
<400> 122
   000
<210> 123
<400> 123
   000
<210> 124
<400> 124
   000
<210> 125
<400> 125
   000
<210> 126
<400> 126
   000
<210> 127
<400> 127
   000
<210> 128
<400> 128
   000
<210> 129
<400> 129
   000
<210> 130
<400> 130
   000
<210> 131
<400> 131
   000
<210> 132
<400> 132
   000
<210> 133
<400> 133
   000
<210> 134
<400> 134
   000
<210> 135
<400> 135
   000
<210> 136
<400> 136
   000
<210> 137
<400> 137
   000
<210> 138
<400> 138
   000
<210> 139
<400> 139
   000
<210> 140
<400> 140
   000
<210> 141
<400> 141
   000
<210> 142
<400> 142
   000
<210> 143
<400> 143
   000
<210> 144
<400> 144
   000
<210> 145
<400> 145
   000
<210> 146
<400> 146
   000
<210> 147
<400> 147
   000
<210> 148
<400> 148
   000
<210> 149
<400> 149
   000
<210> 150
<400> 150
   000
<210> 151
<400> 151
   000
<210> 152
<400> 152
   000
<210> 153
<400> 153
   000
<210> 154
<400> 154
   000
<210> 155
<400> 155
   000
<210> 156
<400> 156
   000
<210> 157
<400> 157
   000
<210> 158
<400> 158
   000
<210> 159
<400> 159
   000
<210> 160
<400> 160
   000
<210> 161
<400> 161
   000
<210> 162
<400> 162
   000
<210> 163
<400> 163
   000
<210> 164
<400> 164
   000
<210> 165
<400> 165
   000
<210> 166
<400> 166
   000
<210> 167
<400> 167
   000
<210> 168
<400> 168
   000
<210> 169
<400> 169
   000
<210> 170
<400> 170
   000
<210> 171
<400> 171
   000
<210> 172
<400> 172
   000
<210> 173
<400> 173
   000
<210> 174
<400> 174
   000
<210> 175
<400> 175
   000
<210> 176
<400> 176
   000
<210> 177
<400> 177
   000
<210> 178
<400> 178
   000
<210> 179
<400> 179
   000
<210> 180
<400> 180
   000
<210> 181
<400> 181
   000
<210> 182
<400> 182
   000
<210> 183
<400> 183
   000
<210> 184
<400> 184
   000
<210> 185
<400> 185
   000
<210> 186
<400> 186
   000
<210> 187
<400> 187
   000
<210> 188
<400> 188
   000
<210> 189
<400> 189
   000
<210> 190
<400> 190
   000
<210> 191
<400> 191
   000
<210> 192
<400> 192
   000
<210> 193
<400> 193
   000
<210> 194
<400> 194
   000
<210> 195
<400> 195
   000
<210> 196
<400> 196
   000
<210> 197
<400> 197
   000
<210> 198
<400> 198
   000
<210> 199
<400> 199
   000
<210> 200
<400> 200
   000
<210> 201
<400> 201
   000
<210> 202
<400> 202
   000
<210> 203
<400> 203
   000
<210> 204
<400> 204
   000
<210> 205
<400> 205
   000
<210> 206
<400> 206
   000
<210> 207
<400> 207
   000
<210> 208
<400> 208
   000
<210> 209
<400> 209
   000
<210> 210
<400> 210
   000
<210> 211
<400> 211
   000
<210> 212
<400> 212
   000
<210> 213
<400> 213
   000
<210> 214
<400> 214
   000
<210> 215
<400> 215
   000
<210> 216
<400> 216
   000
<210> 217
<400> 217
   000
<210> 218
<400> 218
   000
<210> 219
<400> 219
   000
<210> 220
<400> 220
   000
<210> 221
<400> 221
   000
<210> 222
<400> 222
   000
<210> 223
<400> 223
   000
<210> 224
<400> 224
   000
<210> 225
<400> 225
   000
<210> 226
<400> 226
   000
<210> 227
<400> 227
   000
<210> 228
<400> 228
   000
<210> 229
<400> 229
   000
<210> 230
<400> 230
   000
<210> 231
<400> 231
   000
<210> 232
<400> 232
   000
<210> 233
<400> 233
   000
<210> 234
<400> 234
   000
<210> 235
<400> 235
   000
<210> 236
<400> 236
   000
<210> 237
<400> 237
   000
<210> 238
<400> 238
   000
<210> 239
<400> 239
   000
<210> 240
<400> 240
   000
<210> 241
<400> 241
   000
<210> 242
<400> 242
   000
<210> 243
<400> 243
   000
<210> 244
<400> 244
   000
<210> 245
<400> 245
   000
<210> 246
<400> 246
   000
<210> 247
<400> 247
   000
<210> 248
<400> 248
   000
<210> 249
<400> 249
   000
<210> 250
<400> 250
   000
<210> 251
<400> 251
   000
<210> 252
<400> 252
   000
<210> 253
<400> 253
   000
<210> 254
<400> 254
   000
<210> 255
<400> 255
   000
<210> 256
<400> 256
   000
<210> 257
<400> 257
   000
<210> 258
<400> 258
   000
<210> 259
<400> 259
   000
<210> 260
<400> 260
   000
<210> 261
<400> 261
   000
<210> 262
<400> 262
   000
<210> 263
<400> 263
   000
<210> 264
<400> 264
   000
<210> 265
<400> 265
   000
<210> 266
<400> 266
   000
<210> 267
<400> 267
   000
<210> 268
<400> 268
   000
<210> 269
<400> 269
   000
<210> 270
<400> 270
   000
<210> 271
<400> 271
   000
<210> 272
<400> 272
   000
<210> 273
<400> 273
   000
<210> 274
<400> 274
   000
<210> 275
<400> 275
   000
<210> 276
<400> 276
   000
<210> 277
<400> 277
   000
<210> 278
<400> 278
   000
<210> 279
<400> 279
   000
<210> 280
<400> 280
   000
<210> 281
<400> 281
   000
<210> 282
<400> 282
   000
<210> 283
<400> 283
   000
<210> 284
<400> 284
   000
<210> 285
<400> 285
   000
<210> 286
<400> 286
   000
<210> 287
<400> 287
   000
<210> 288
<400> 288
   000
<210> 289
<400> 289
   000
<210> 290
<400> 290
   000
<210> 291
<400> 291
   000
<210> 292
<400> 292
   000
<210> 293
<400> 293
   000
<210> 294
<400> 294
   000
<210> 295
<400> 295
   000
<210> 296
<400> 296
   000
<210> 297
<400> 297
   000
<210> 298
<400> 298
000
<210> 299
<400> 299
   000
<210> 300
<400> 300
   000
<210> 301
<400> 301
   000
<210> 302
<400> 302
   000
<210> 303
<400> 303
   000
<210> 304
<400> 304
   000
<210> 305
<400> 305
   000
<210> 306
<400> 306
   000
<210> 307
<400> 307
   000
<210> 308
<400> 308
   000
<210> 309
<400> 309
   000
<210> 310
<400> 310
   000
<210> 311
<400> 311
   000
<210> 312
<400> 312
   000
<210> 313
<400> 313
   000
<210> 314
<400> 314
   000
<210> 315
<400> 315
   000
<210> 316
<400> 316
   000
<210> 317
<400> 317
   000
<210> 318
<400> 318
   000
<210> 319
<400> 319
   000
<210> 320
<400> 320
   000
<210> 321
   <211> 1333
   <212> DNA
   <213> Homo sapiens
<400> 321
   gcccacgcgt ccgatggcgt tcacgttcgc ggccttctgc tacatgctgg 50
   cgctgctgct cactgccgcg ctcatcttct tcgccatttg gcacattata 100
   gcatttgatg agctgaagac tgattacaag aatcctatag accagtgtaa 150
   taccctgaat ccccttgtac tcccagagta cctcatccac gctttcttct 200
   gtgtcatgtt tctttgtgca gcagagtggc ttacactggg tctcaatatg 250
   cccctcttgg catatcatat ttggaggtat atgagtagac cagtgatgag 300
   tggcccagga ctctatgacc ctacaaccat catgaatgca gatattctag 350
   catattgtca gaaggaagga tggtgcaaat tagcttttta tcttctagca 400
   tttttttact acctatatgg catgatctat gttttggtga gctcttagaa 450
   caacacacag aagaattggt ccagttaagt gcatgcaaaa agccaccaaa 500
   tgaagggatt ctatccagca agatcctgtc caagagtagc ctgtggaatc 550
   tgatcagtta ctttaaaaaa tgactcctta ttttttaaat gtttccacat 600
   ttttgcttgt ggaaagactg ttttcatatg ttatactcag ataaagattt 650
   taaatggtat tacgtataaa ttaatataaa atgattacct ctggtgttga 700
   caggtttgaa cttgcacttc ttaaggaaca gccataatcc tctgaatgat 750
   gcattaatta ctgactgtcc tagtacattg gaagcttttg tttataggaa 800
   cttgtagggc tcattttggt ttcattgaaa cagtatctaa ttataaatta 850
   gctgtagata tcaggtgctt ctgatgaagt gaaaatgtat atctgactag 900
   tgggaaactt catgggtttc ctcatctgtc atgtcgatga ttatatatgg 950
   atacatttac aaaaataaaa agcgggaatt ttcccttcgc ttgaatatta 1000
   tccctgtata ttgcatgaat gagagatttc ccatatttcc atcagagtaa 1050
   taaatatact tgctttaatt cttaagcata agtaaacatg atataaaaat 1100
   atatgctgaa ttacttgtga agaatgcatt taaagctatt ttaaatgtgt 1150
   ttttatttgt aagacattac ttattaagaa attggttatt atgcttactg 1200
   ttctaatctg gtggtaaagg tattcttaag aatttgcagg tactacagat 1250
   tttcaaaact gaatgagaga aaattgtata accatcctgc tgttccttta 1300
   gtgcaataca ataaaactct gaaattaaga ctc 1333
<210> 322
   <211> 144
   <212> PRT
   <213> Homo sapiens
<400> 322
   Met Ala Phe Thr Phe Ala Ala Phe Cys Tyr Met Leu Ala Leu Leu
   1 5 10 15
   Leu Thr Ala Ala Leu Ile Phe Phe Ala Ile Trp His Ile Ile Ala
   20 25 30
   Phe Asp Glu Leu Lys Thr Asp Tyr Lys Asn Pro Ile Asp Gln Cys
   35 40 45
   Asn Thr Leu Asn Pro Leu Val Leu Pro Glu Tyr Leu Ile His Ala
   50 55 60
   Phe Phe Cys Val Met Phe Leu Cys Ala Ala Glu Trp Leu Thr Leu
   65 70 75
   Gly Leu Asn Met Pro Leu Leu Ala Tyr His Ile Trp Arg Tyr Met
   80 85 90
   Ser Arg Pro Val Met Ser Gly Pro Gly Leu Tyr Asp Pro Thr Thr
   95 100 105
   Ile Met Asn Ala Asp Ile Leu Ala Tyr Cys Gln Lys Glu Gly Trp
   110 115 120
   Cys Lys Leu Ala Phe Tyr Leu Leu Ala Phe Phe Tyr Tyr Leu Tyr
   125 130 135
   Gly Met Ile Tyr Val Leu Val Ser Ser
   140
<210> 323
   <211> 477
   <212> DNA
   <213> Homo sapiens
<400> 323
   attatagcat ttgatgagct gaagactgat tacaagatcc tatagaccag 50
   tgtaataccc tgaatcccct tgtactccca gagtacctca tccacgcttt 100
   cttctgtgtc atgtttcttt gtgcagcaga gtggcttaca ctgggtctca 150
   atatgcccct cttggcatat catatttgga ggtatatgag tagaccagtg 200
   atgagtggcc caggactcta tgaccctaca accatcatga atgcagatat 250
   tctagcatat tgtcagaagg aaggatggtg caaattagct ttttatcttc 300
   tagcattttt ttactaccta tatggcatga tctatgtttt ggtgagctct 350
   tagaacaaca cacagaagaa ttggtccagt taagtgcatg caaaaagcca 400
   ccaaatgaag ggattctatc cagcaagatc ctgtccaaga gtagcctgtg 450
   gaatctgatc agttacttta aaaaatg 477
<210> 324
   <211> 43
   <212> DNA
   <213> Artificial
<220>
   <223> Artificial Sequence
<400> 324
   tgtaaaacga cggccagtta aatagacctg caattattaa tct 43
<210> 325
   <211> 41
   <212> DNA
   <213> Artificial
<220>
   <223> Artificial Sequence
<400> 325
   caggaaacag ctatgaccac ctgcacacct gcaaatccat t 41
<210> 326
   <211> 20
   <212> DNA
   <213> Artificial
<220>
   <223> Artificial Sequence
<400> 326
   gtgcagcaga gtggcttaca 20
<210> 327
   <211> 20
   <212> DNA
   <213> Artificial
<220>
   <223> Artificial Sequence
<400> 327
   actggaccaa ttcttctgtg 20
<210> 328
   <211> 45
   <212> DNA
   <213> Artificial
<220>
   <223> Artificial Sequence
<400> 328
   gatattctag catattgtca gaaggaagga tggtgcaaat tagct 45